(19) Europäisches Patentamt / European Patent Office / Office européen des brevets

(11) EP 2 356 977 B1

(12) EUROPÄISCHE PATENTSCHRIFT

(45) Veröffentlichungstag und Bekanntmachung des Hinweises auf die Patenterteilung:
**27.12.2017 Patentblatt 2017/52**

(51) Int Cl.:
***A61K 8/34*** *(2006.01)* ***A61K 8/41*** *(2006.01)*
***A61K 8/49*** *(2006.01)* ***A61K 8/97*** *(2017.01)*
***A61Q 19/06*** *(2006.01)* ***A61K 36/483*** *(2006.01)*

(21) Anmeldenummer: **11153073.9**

(22) Anmeldetag: **02.02.2011**

(54) **Zubereitungen mit Holzextrakten von Gleditschien**

Preparations with wood extracts of Gleditsia

Préparations dotées d’extraits de bois de Gleditsia

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.08.2011 Patentblatt 2011/33**

(73) Patentinhaber: **Symrise AG**
**37603 Holzminden (DE)**

(72) Erfinder:
- **Herrmann, Martina**
  **31789, Hameln (DE)**
- **Joppe, Holger**
  **37586, Dassel (DE)**
- **Meyer, Imke**
  **37619, Bodenwerder (DE)**
- **Schaper, Karin**
  **37627, Linnenkamp (DE)**
- **Küper, Thomas**
  **48734, Reken (DE)**
- **Betke, Julia**
  **32694, Dörentrup (DE)**

(74) Vertreter: **Fabry, Bernd et al**
**IP2 Patentanwalts GmbH**
**Schlossstrasse 523-525**
**41238 Mönchengladbach (DE)**

(56) Entgegenhaltungen:
**EP-A1- 1 541 127**
**WO-A1-2010/048114**
**DE-A1-102004 032 837**
**FR-A1- 2 740 681**
**FR-A1- 2 799 121**
**US-A1- 2002 106 388**

- **BRANDEKOW RJ: "Present and potential sources of tannin in the united states", JOURNAL OF FORESTRY,, Bd. 45, Nr. 10, 1. Oktober 1947 (1947-10-01), Seiten 729-734, XP009149727,**
- **CHADENSON M MOLHO-LACROIX ET AL: "Sur les constiuants flavoniques du fevier (Gleditschia triacanthos)", COMPTES RENDUS HEBDOMADAIRES DES SEANCES DE L’ACADEMIE DESSCIENCES, GAUTHIER-VILLARS, PARIS, FR, Bd. 240, 1. Januar 1955 (1955-01-01), Seiten 1362-1364, XP009149732, ISSN: 0001-4036**
- **WIKIPEADIA: "Fisetin", 20110222, [Online] 22. Februar 2011 (2011-02-22), Seiten 1-3, XP007918977, Gefunden im Internet: URL:http://en.wikipedia.org/wiki/Fisetin> [gefunden am 2011-06-29]**
- **HOWITZ K T ET AL: "Small molecule activators of sirtuins extend Saccharomyces cerevisiae lifespan", NATURE, NATURE PUBLISHING GROUP, LONDON, GB, Bd. 425, Nr. 6954, 11. September 2003 (2003-09-11), Seiten 191-196, XP002379361, ISSN: 0028-0836, DOI: DOI:10.1038/NATURE01960**
- **ROWE J ET AL: "Extractives in Eastern Hardwoods - A Review", GENERAL TECHNICAL REPORT FPL 18,, 1 January 1979 (1979-01-01), XP007918985,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 356 977 B1

**Beschreibung**

Die vorliegende Erfindung betrifft primär die Verwendung von Gleditschie (*Gleditsia*) Holz- bzw. Kernholzextrakten als anti-cellulite Wirkstoffe. Die vorliegende Erfindung betrifft ferner bestimmte Gleditschie Holzextrakte und Mischungen enthaltend Gleditschie Holzextrakte sowie entsprechende kosmetische, dermatologische oder pharmazeutische Zubereitungen, die sich insbesondere zur Prophylaxe und Behandlung von Cellulite beim Menschen eignen.

Der normale Alterungsprozess wird allgemein als die Veränderung des Körpers im Lauf des Lebens angesehen. Generell wird unterschieden zwischen intrinsischer (genetisch bedingter) und extrinsischer (umweltbedingter) Alterung. Zu den exogenen Faktoren, die zu einer vorzeitigen Hautalterung (sogenannte Lichtalterung) führen, gehören vor allem UV-Exposition, Genussgifte (Tabak, Alkohol) und Klima- bzw. Witterungseinflüsse. Mit zunehmendem Alter kommt es bei Erwachsenen zu einem kontinuierlichen Verlust organspezifischer Funktionen, der mit verschiedenen Veränderungen wie z.B. einer Verringerung der metabolischen Aktivität, einer geringen Zellteilungsrate, einer verringerten DNA-Reparatur Kapazität, einer Versteifung von Gefäßwänden und einer beeinträchtigten peripheren Blutzirkulation einhergeht. All diese Aspekte sind eng miteinander verbunden.

Im Rahmen der Alterung kommt es auch zu ausgeprägten strukturellen Veränderungen der Haut. Sie spielen sich auf zellulärer und nichtzellulärer Ebene ab, erfassen alle Schichten der Haut und sind sowohl mikro- als auch makromorphologisch sichtbar. Die Epidermis (Oberhaut) wird aufgrund einer Verringerung der Mitoserate der Basalzellen dünner. Zusätzlich können höherer Wasserverlust durch Diffusion, eine verringerte Elastizität, eine gröbere äußere Struktur und Faltenbildung beobachtet werden. Die gealterte Dermis (Lederhaut) ist gekennzeichnet durch weniger Fibroblasten mit geringerer Aktivität. Zusätzlich kann eine verringerte Produktion von Tropocollagen, Hyaluronsäure und Elastin beobachtet werden sowie reduzierte Talg- und Schweißdrüsensekretion. Die Subkutis (Unterhautfettgewebe) verliert an Volumen.

Eine Kalorienrestriktion, d.h. eine um 20 bis 50% reduzierte Nährstoffaufnahme, führt bei verschiedenen Organismen, darunter auch Säugetiere, zu einer verzögerten Alterung und hat gesundheitsfördernde Wirkung. So reduziert oder verschiebt sich das Auftreten altersbedingter Erkrankungen wie z.B. Diabetes und kardiovaskuläre Erkrankungen und ein verbesserter Schutz gegenüber zum Beispiel Hitze und oxidativem Stress wird induziert.

Cellulite - auch unter den Synonyma Protrusio cutis und umgangssprachlich als Orangenhaut bekannt - stellt ein kosmetisch-ästhetisches Problem dar, das mit Grübchenbildung und Vertiefungen der Haut und Knötchenbildung des subkutanen Fettgewebes einhergeht. Cellulite kann an jeder Körperstelle des Menschen auftreten, am häufigsten sind jedoch die Aussen- und Rückseite der Oberschenkel sowie das Gesäß betroffen. Auch Brüste, Unterbauch, Oberarme oder Nacken sind manchmal durch Cellulite geprägt.

Cellulite kann zwar regelmäßig an menschlichen Körperstellen mit übermäßiger Fettablagerung gefunden werden, doch stellt das Übergewicht keine Voraussetzung für deren Auftreten dar. Vermehrt haben auch schlanke Frauen ausgeprägte Cellulite-Erscheinungen. Zwischen der Schwere der Ausprägung der Cellulite und dem prozentualen Fettanteil im Gewebe besteht allerdings wohl eine Korrelation.

Die geschlechtsspezifische anatomische Struktur der Haut des Menschen hat einen großen Einfluss auf die Entstehung der Cellulite. So ist beim Mann Cellulite nur selten zu beobachten, dagegen sind 80% - 90% aller Frauen betroffen. Insbesondere der Aufbau der Dermis wirkt sich auf das Hautrelief aus. So werden beim Mann die Fettkammern beim Zusammenpressen der Haut durch sich überkreuzende Bindegewebssepten und den damit verbundenen klammerartigen Einschluss der Fettzellen zurückgehalten. Dagegen wölben sich bei der Frau die röhrenförmig voneinander getrennten Fettkammern, die durch radiär verlaufende Bindegewebssepten eingeschlossen sind, beim Zusammenpressen hoch.

Die herkömmlichen Behandlungsmethoden der Cellulite versuchen die Durchblutung der betroffenen Hautpartien zu fördern und die Bindegewebsstruktur positiv zu beeinflussen, beispielsweise durch Massage, Lymphdrainage, Diät, Sport, Magnetfelder oder auch Liposuction (Fettabsaugung).

Mit Cellulite einhergehen kann zudem sensorisches Missempfinden, wie Hautreizungen und Hautentzündungen, der betroffenen Körperstellen.

Unter Hautentzündung ist im Zusammenhang mit dieser Anmeldung jede Veränderung der Haut zu verstehen, die beim Menschen sensorisches Missempfinden auslöst oder/und durch ein trockenes, gerötetes und/oder entzündetes Hautbild geprägt ist. Dabei umfasst der Begriff "sensorisches Missempfinden" selbstverständlich auch Zustände wie Jucken oder Schmerz. Hautirritation kann insbesondere phänomenologisch verschiedene Hautzustände umfassen: sensible Haut, empfindliche Haut (inklusive empfindlicher Kopfhaut), verletzliche Haut, atopische Haut, irritierte Haut, entzündete Haut, die sich im jeweils höheren Schweregrad in einer Hautrötung, dem sogenannten Erythem äußert.

Das Problem "sensible Haut" betrifft eine wachsende Anzahl Erwachsener und Kinder. Man geht inzwischen davon aus, dass bis zu 50 % der Bevölkerung eine sensible Haut haben. Mit sensibler Haut bezeichnet man eine Haut mit einer erniedrigten Reizschwelle für Irritantien, wie hyperreaktive und intolerante, aber auch atopische Haut. Bei Menschen mit sensibler, empfindlicher oder verletzlicher Haut kann ein mit "Stinging" (engl. "to sting" = verletzen, brennen, schmerzen) bezeichnetes Phänomen beobachtet werden. Typische, mit den Begriffen "Stinging" oder "empfindlicher Haut" in Verbindung gebrachte, störende Phänomene sind Hautrötung, Kribbeln, Prickeln, Spannen und Brennen der Haut und Juckreiz. Sie können durch stimulierende Umgebungsbedingungen wie z. B. Massage, Tensideinwirkung, Wettereinfluss wie Wärme, Kälte, Trockenheit, aber auch feuchte Wärme, Wärmestrahlung und UV-Strahlung, z. B. der Sonne, oder psychologischen Stress hervorgerufen werden.

"Empfindliche" Kopfhaut ist ebenfalls gekennzeichnet durch Hautrötung, Kribbeln, Prickeln, Brennen und Juckreiz. Auslöser sind beispielsweise Seife, Shampoos oder andere Haarpflegemittel, Tenside, hartes Wasser mit hohen Kalkkonzentrationen und/oder mechanische Beanspruchung. Erytheme und Hyperseborrhoe (übermäßige Talgproduktion) der Kopfhaut sowie Schuppen sind häufig mit den beschriebenen Phänomenen assoziiert.

Bei ca. 10-20% der Bevölkerung industrieller Länder, mit steigender Tendenz, ist die Atopie zu beobachten, eine familiär auftretende Überempfindlichkeit der Haut und der Schleimhäute gegen Umweltstoffe, mit gesteigerter Bereitschaft, gegen Substanzen aus der natürlichen Umwelt Überempfindlichkeitsreaktionen vom Soforttyp (Allergien) zu entwickeln. Atopie ist vermutlich genetisch bedingt. Atopie kann sich als atopische Dermatitis äußern. Dabei ist die Hautbarriere geschädigt, die Haut ist oft entzündet und juckt.

Sirtuine (Sirtuin Proteine) sind eine Klasse von NAD+-abhängigen Histon- und Protein-Deacetylasen, die unter Kalorienrestriktion hochreguliert werden. Für Säugetiere, d.h. auch für den Menschen, wurden bisher 7 Sirtuine (SIRT1-7) beschrieben, von diesen ist SIRT1 das am besten untersuchte. Die Lokalisierung in den Zellen ist für die 7 humanen Sirtuine sehr unterschiedlich, so sind offenbar SIRT6 und SIRT7 im Zellkern, SIRT1 überwiegend im Zellkern aber auch im Cytoplasma, SIRT2 meist im Cytoplasma aber auch im Zellkern und SIRT3, SIRT4 sowie SIRT5 ausschließlich in den Mitochondrien lokalisiert. Sirtuine deacetylieren als Histon-Deacetylasen (HDAC) der Klasse III, die Histonproteine über einen NAD+-abhängigen Mechanismus. Diese und andere posttranslationale Veränderungen an den Seitenketten einzelner Aminosäuren von Histonproteinen tragen dazu bei, die Aktivierung und Stilllegung von bestimmten Genabschnitten zu regulieren. Solche Kontrollmechanismen der Genexpression jenseits der DNA-Sequenz, die nicht verändert wird, werden als Epigenetik bezeichnet.

Sirtuine deacetylieren darüber hinaus auch einer Reihe wichtiger Nicht-Histonproteine, darunter unter anderem bedeutende Transkriptionsfaktoren wie z.B. p53, PGC-1alpha, NF-κB, p300 und Transkriptionsfaktoren aus der FOXO-Gruppe und regulieren dadurch ihre Aktivität. Diese Proteinsubstrate gehören zu den zentralen Regulatoren des zellulären Metabolismus und Energiehaushalts, und spielen eine wichtige Rolle bei z.B. Entzündungen und der zellulären Stressantwort.

Der entzündungshemmende Effekt von SIRT basiert unter anderem auf Unterdrückung der NF-κB Aktivität und/oder auf Cyclooxygenase-2 (COX-2) Hemmung. Eine überhöhte Aktivierung von NF-κB wurde für Altershaut beschrieben und verbindet so Inflammation mit Alterung. Eine Auswirkung der NF-κB Aktivierung ist die Expression von Matrix-Metalloproteinasen (MMPs), Enzymen die verschiedene Faserproteine der Extrazellularmatrix wie Kollagen und Elastin abbauen.

Die erythematöse Wirkung des ultravioletten Teils der Sonnenstrahlung oder künstlicher Strahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet und beeinträchtigt das Wohlbefinden der Betroffenen selbst in leichten Fällen.

Kosmetische Aktivatoren von SIRT bieten sich daher einerseits als Wirkstoffe gegen Alterung, insbesondere als Wirkstoffe gegen Hautalterung (im Rahmen dieses Textes auch als "anti-aging Wirkstoffe" bezeichnet), und als generelle zelluläre Stressprotektoren an. Weiterhin bieten sie Vorteile als entzündungshemmende Wirkstoffe und verhindern das durch Entzündungen bedingte Altern (inflammaging) indem sie proinflammatorische Zytokine reduzieren, was zu einem gleichmäßigen Hautton führt und Pigmentflecken reduziert.

Die Regulierung des Fettstoffwechsels im Fettgewebe des Menschen zur Reduzierung der eingelagerten Lipidmenge kann prinzipiell auf drei Wegen erfolgen:

Weg (i) - Adipogenese: Die Differenzierung der als Preadipozyten bezeichneten Vorläuferzellen der Fettzellen zu den eigentlichen Fettzellen (Adipozyten genannt), die Triglyceride einlagern können, kann gehemmt werden. Vereinfacht ausgedrückt verhindert eine Hemmung des Wegs (i) den Aufbau der Cellulite dadurch, dass die Anzahl der Fettzellen nicht zunimmt.

Weg (ii) - Lipogenese: Die Einlagerung von Triglyceriden in den Adipozyten kann gehindert bzw. inhibiert werden. Vereinfacht ausgedrückt verhindert eine Hemmung des Wegs (ii) die Einlagerung weiterer Triglyceride (Fette) in der Zelle, vorhandene Fettzellen lagern kein neues Fett ein. Durch den natürlichen Fettstoffwechsel nimmt bei Hemmung des Wegs (ii) der Fettgehalt in der Zelle ab.

Weg (iii) - Lipolyse: Eine vermehrte / verstärkte Hydrolyse bereits in den Adipozyten eingelagerter Lipide ist durch eine gezielte Stimulation möglich. Dadurch erfolgt eine Reduzierung der in subkutanem Fettgewebe bereits enthaltenen Lipidmenge. Vereinfacht ausgedrückt verstärkt eine Stimulierung des Wegs (iii) den Abbau der bereits in der Zelle vorhandenen Fette, eine hemmende / inhibierende, d.h. antagonistische, Wirkung bezüglich des Wegs (iii) hingegen be- bzw. verhindert den Fettabbau.

Die Differenzierung von Zellen ist die Veränderung der Regulierung der Genaktivität einer Zelle, so dass über Transkription und Protein-Biosynthese verschiedene Protein-Bestände in den Zellen realisiert werden und sich die Zellen nach Aussehen und Funktion unterscheiden. So exprimieren Adipozyten erst nach der Differenzierung Enzyme, die für die Einlagerung von Fetten notwendig sind. In ihren Vorläuferzellen undifferenzierten Preadipozyten werden diese Enzyme nicht oder nur in sehr geringem Umfang exprimiert.

Kosmetische Zubereitungen, welche die Prophylaxe und Behandlung von Cellulite zum Ziel haben, sind in der Literatur bereits vorgeschlagen worden.

In EP 1 234 572 wird eine kosmetische Zubereitung aus mindestens einem Isoflavon-Aglykon aus der Gruppe Genistein, Daidzein, Glycitein, Formononetin, Tectorigenin, Irigenin, Biochanin A, O-Desmethylangolensin, Equol, Orobol, Santal, Pratensin und Apiosylpuerarin, insbesondere Genistein und/oder Daidzein, zur Behandlung von Cellulite beschrieben.

In DE 10 2004 032 837 wird eine kosmetische Zubereitung aus bestimmten Biochinonen und Isoflavonoiden, bevorzugt Genistein, zur Prophylaxe von Cellulite beschrieben. Es wird behauptet, dass die Wirkung dieser Zubereitung über eine Verbesserung des Zellstoffwechsels erfolgt. Es ist dabei nicht ersichtlich, welcher Mechanismus des Zellstoffwechsels verbessert wird.

WO 2010/048114 beschreibt Naturstoffe zur Modifizierung der Adipozytenphysiologie.

Journal of Biochemistry 2004, 135(1), 85-91 beschreibt den inhibierenden Effekt von Fisetin auf die Fettsäure-Synthase.

Laut Biochem. Pharmacol. 1992, 44, 1307-1315 wird die Lipolyse nicht grundsätzlich durch Phosphodiesterase-Inhibitoren stimuliert, da die Lipolyse durch verschiedene Faktoren beeinflusst wird.

WO 2005/002672 eine Reihe von Verbindungen an, die die Sirtuin Enzymaktivität stimulieren darunter auch natürlich vorkommende Substanzen u.a. Resveratrol, dem wohl meistgenannten Stimulator der SIRT1-Enzymaktvität, Flavonoide wie Fisetin, Luteolin, 3,6,3',4'-Tetrahydroxyflavon, Quercetin, Naringin und 3,5,7,3',4'-Pentahydroxyflavanon. Allerdings erscheint fraglich inwieweit der in WO 2005/002672 genutzte in-vitro Test zur Bestimmung der Stimulierung der SIRT1-Enzymaktivität unter Nutzung eines synthetischen, artifiziellen acylierten Peptidsubstrats überhaupt die wirkliche Situation widerspiegelt, da an tatsächlichen biologischen Substraten kein stimulierender Effekt durch Resveratrol festgestellt werden konnte (Chem. Biol. Drug Des. 2009, 74, 619-624). Auch kann von einer stimulierenden Wirkung auf die Sirtuin-Enzymaktivität nicht auf eine gleichzeitige Stimulierung der Sirtuin-Proteinexpression geschlossen werden.

Es war die primäre Aufgabe der vorliegenden Erfindung, Mittel mit anti-cellulite Wirksamkeit anzugeben. Die anzugebenden Mittel sollten in zahlreichen unterschiedlichen Zubereitungen, insbesondere in kosmetischen, dermatologischen und pharmazeutischen Zubereitungen, einsetzbar sein. Vorzugsweise sollten die Mittel natürlichen Ursprungs, leicht herstellbar und gut lagerfähig sein. Darüber hinaus sollten Herstellverfahren für entsprechende Mittel und deren Verwendungen angegeben werden.

Es wäre von Vorteil, wenn das gesuchte Mittel zudem positiv auf Hautreizungen wirken würde, die mit einer Cellulite einhergehen können.

Überraschenderweise hat sich in umfangreichen eigenen Untersuchungen gezeigt, dass *Gleditsia* Holzextrakte sich hervorragend als anti-cellulite Wirkstoffe eignen.

In einem ersten Aspekt betrifft die vorliegende Erfindung die nicht-therapeutische Verwendung eines Holzextraktes von *Gleditsia triacanthos*

i) zur, Vorbeugung, Behandlung oder Verringerung von Cellulite,
und/oder
(ii) zum nicht-therapeutischen

- Hemmen der Differenzierung von Preadipozyten,
und/oder

- Hemmen der Lipogenese in Adipozyten,
und/oder

- Reduzieren der in subkutanem Fettgewebe enthaltenen Lipidmenge.

Die erfindungsgemäßen bzw. erfindungsgemäß einzusetzenden *Gleditsia* Holzextrakte zeigen zusätzlich SIRT-stimulierende, d.h. SIRT-aktivierende, Wirksamkeit, insbesondere bezüglich SIRT1.

Insbesondere die Erhöhung des SIRT1 Proteinlevels, d.h. des SIRT1 Proteingehaltes, oder der Enzymaktivität im weißen adiposen Gewebe führt dabei vermutlich zu einer weiter verminderten Adipogenese sowie einer geringeren Lipideinlagerung. Auch aufgrund dieser Eigenschaften wirken die erfindungsgemäßen bzw. erfindungsgemäß einzusetzenden *Gleditsia* Holzextrakte als Wirkstoffe für die Verhinderung und Reduzierung von Cellulite.

Die erfindungsgemäßen bzw. erfindungsgemäß einzusetzenden *Gleditsia* Holzextrakte sind ferner anti-irritierende Wirkstoffe.

Insgesamt zeigen die erfindungsgemäßen bzw. erfindungsgemäß einzusetzenden *Gleditsia* Holzextrakte neben der gesuchten anti-cellulite - Wirksamkeit zusätzlich SIRT-stimulierende / -aktivierende und anti-irritierende Wirksamkeit. Die erfindungsgemäßen bzw. erfindungsgemäß einzusetzenden *Gleditsia* Holzextrakte sind dadurch als anti-aging Wirkstoffe, insbesondere als Wirkstoffe gegen Hautalterung, einsetzbar.

Erfindungsgemäß bevorzugt ist die Verwendung eines Extrakt des Holzes, vorzugsweise des Kernholzes, von *Gleditsia triacanthos,* da mit diesen besonders gute Ergebnisse erzielt werden konnten.

Die Gleditschien (*Gleditsia*), auch Lederhülsenbäume genannt, sind eine Pflanzengattung aus der Familie der Hülsenfrüchtler (Fabaceae). Sie sind in den gemäßigten und subtropischen Regionen Nord- und Südamerikas sowie in Teilen des gemäßigten und subtropischen Asiens und im tropischen Afrika beheimatet.

*Gleditsia triacanthos,* auch bekannt als Amerikanische Gleditschie oder (Falscher) Christusdorn, ist ein in den zentralen und östlichen Teilen der USA beheimateter sommergrüner, bis 20 m hoher Baum. Der Stamm ist dornenbewehrt mit großen Büscheln starker, verzweigter Dornen, die bis 30 cm lang sind, was zu der Bezeichnung (Falscher) Christusdorn führte. Auffällig sind die Hülsenfrüchte, die im Herbst und Winter als etwa 25 (in warmen Lagen auch bis zu 50) Zentimeter lange und 2,5 bis 4 Zentimeter breite Hülsen von den Bäumen herabhängen. Sie sind spiralig gedreht und werden später braun. In Mitteleuropa ist die *Gleditsia triacanthos* völlig winterhart und wird dort häufig in Parks und Anlagen gepflanzt.

Die Samen des Lederhülsenbaumes sind roh oder gekocht essbar und enthalten große Mengen an Polysacchariden. Für diese beschreibt FR 2067649 die kosmetische Verwendung als Haar weichmachenden und entkräuselnden Wirkstoff und GB 1,065,910 die Verwendung als Hilfsstoff für Arzneimittel.

Die Frucht (CN 101317960) bzw. ein ethanolischer Fruchtextrakt (Int. J. Mol. Med. 2009, 23(1):121-9) sowie die Dornen (CN 101224287) der chinesischen Gleditschie (*G. sinensis*) werden, zum Teil in Kombination mit anderen Pflanzen(teilen) der traditionellen chinesischen Medizin, als anti-rheumatisch, anti-arthritisch, anti-inflammatorisch und Cyclooxygenase-2 Hemmer beschrieben.

Die japanische Gleditschie (*G. japonica*) wird als Teil von Zusammensetzungen mit Haarwuchs fördernder, Schuppen und Juckreiz hemmender Wirksamkeit (JP 2001288047 und JP 2000044439) sowie mit Haarergrauen verhindernder Aktivität (JP 2001131026) beschrieben.

In Yakugaku Zasshi 1957, 77, 1208-1210 wurden Holzraspat des Kernholzes von *G. japonica* mit Methanol bzw. Ethylacetat extrahiert und analysiert. In den Extrakten wurden Fisetin, Fustin und Gledistin als Inhaltsstoffe des Kernholzes von *G. japonica* identifiziert.

Compt. Rend. Acad. Sci., Paris, 1955, 240, 1362-1364 beschreibt die erschöpfende Extraktion von *Gleditsia triacanthos* Holzpulver mit Ether in der Kälte und die anschließende Isolierung von Fustin und Fisetin.

Die Zusammensetzung eines Pflanzenteils einer Art einer Gattung ist nicht unbedingt auf die Zusammensetzung einer anderen Art derselben Gattung übertragbar.

Weiterhin hängen die Inhaltsstoffe und damit die biologische Aktivität von Extrakten in der Regel sehr stark von dem jeweils extrahierten Pflanzenteil ab. Letzteres zeigt beispielsweise Yakugaku Zasshi 1957, 77 für die Substanzklasse der Saponine, einem Hauptbestandteil der Früchte von *Gleditsia japonica.* Diese Saponine wurden nur im Mesocarp der Früchte, nicht aber in anderen Teilen der Frucht oder anderen Teilen der Pflanze wie Rinde oder Dornen gefunden. Ferner spielen auch die Extraktionsbedingungen, insbesondere das eingesetzte Extraktionsmittel, eine Rolle.

Das Holz bzw. Kernholz von *Gleditsia triacanthos* ist sehr dauerhaft, grüngelblich bis rotbraun gefärbt und hart. Es wird lokal für die Herstellung von Paletten und Kisten sowie allgemein für den Möbel- und Innenausbau genutzt. Hinweise auf eine topische oder orale Verwendung, insbesondere eine topische oder orale kosmetische, dermatologische oder pharmazeutische Verwendung von Holz- bzw. Kernholzextrakten von Gleditsia *triacanthos* sind nicht beschrieben.

Kernholz bezeichnet im Stammquerschnitt die physiologisch nicht mehr aktive, meist dunklere, innere Zone, die sich deutlich vom äußeren, hellen Splintholz unterscheidet.

Erfindungsgemäß wird ein Extrakt aus Holz, vorzugsweise aus Kernholz, von *Gleditsia triacanthos,* verwendet.

Als Trockenextrakt wird im Rahmen des vorliegenden Textes ein Extrakt aus Holz, vorzugsweise Kernholz, von Pflanzen der Gattung *Gleditsia triacanthos* bezeichnet, vorzugsweise hergestellt oder herstellbar nach dem nachfolgend beschriebenen erfindungsgemäßen Verfahren, nach vollständiger Entfernung des Wassers und des Extraktionsmittels bzw. der Extraktionsmittel (für den Fall, dass mehrere Extraktionsmittel verwendet werden).

Ein erfindungsgemäß geeignetes Verfahren zum Herstellen eines Extraktes aus *Gleditsia* Holz bzw. Kernholz

umfasst folgende Schritte:

(1) Bereitstellen von Holz, vorzugsweise von Holzraspat, von *Gleditsia triacanthos,*

(2) Versetzen des in Schritt (1) bereitgestellten Holzes mit einem Extraktionsmittel ausgewählt aus der Gruppe bestehend aus Wasser, Methanol, Ethanol, n-Propanol, Isopropanol, Aceton, Essigester, überkritischem Kohlendioxid und Mischungen zweier oder mehrerer dieser Extraktionsmittel,

(3) bis zu 24 h Extrahieren des in Schritt (2) mit dem Extraktionsmittel versetzten Holzes zum Gewinnen eines Holzextraktes, und

(4) gegebenenfalls partielles oder vollständiges Entfernen des oder der in Schritt (2) verwendeten Extraktionsmittel.

Die Erfindung betrifft ferner neue Extrakte hergestellt oder herstellbar mit einem Verfahren umfassend oder bestehend aus den Schritten:

(1) Bereitstellen von Holz, vorzugsweise von Holzraspat, bevorzugt von Kernholzraspat, von *Gleditsia triacanthos,*

(2) Versetzen des in Schritt (1) bereitgestellten Holzes mit einem Extraktionsmittel ausgewählt aus der Gruppe bestehend aus Wasser, Methanol, Ethanol, n-Propanol, Isopropanol, Aceton, Essigester, überkritischem Kohlendioxid und Mischungen zweier oder mehrerer dieser Extraktionsmittel,

(3) bis zu 24 h Extrahieren des in Schritt (2) mit dem Extraktionsmittel versetzten Holzes zum Gewinnen eines Holzextraktes, und

(4) gegebenenfalls partielles oder vollständiges Entfernen des oder der in Schritt (2) verwendeten Extraktionsmittel,

mit der Maßgabe, dass die durch Extraktion des Kernholzes von *Gleditsia japonica* mit Methanol oder mit Ethylacetat (Essigester) erhaltene Extrakte ausgenommen sind.

Dabei werden als Holzraspat vorzugsweise gewählt: Holzschnitzel, Holzspäne, beispielsweise Hobelspäne, und/oder Holzmehl, beispielsweise Sägemehl, und/oder auf andere Weise geraspeltes oder zerkleinertes Holz.

Zur Herstellung eines erfindungsgemäßen bzw. erfindungsgemäß einzusetzenden *Gleditsia* Holzextrakts wird das Verhältnis der Masse von Extraktionsmittel zu Holzmasse vorzugsweise so eingestellt, dass zumindest die 2-fache Masse an Extraktionsmittel und vorzugsweise nicht mehr als die 30-fache Masse an Extraktionsmittel beträgt, jeweils bezogen auf die eingesetzte Holzmasse. Bevorzugt liegt das Massenverhältnis von Extraktionsmittel zu Holzmasse im Bereich von 3 : 1 bis 25 : 1, besonders bevorzugt im Bereich von 4 : 1 bis 20 : 1, weiter bevorzugt im Bereich von 5 : 1 bis 15 : 1.

Die Extraktionszeit zum Durchführen von Schritt (3) beträgt erfindungsgemäß vorzugsweise höchstens 24 Stunden. Es ist bevorzugt, das Holzraspat in Schritt b) zumindest 1 h lang, insbesondere zumindest 2 h lang zu extrahieren. Die zum Herstellen eines Extrakts für die Verwendung in kosmetischen, dermatologischen oder pharmazeutischen Zubereitungen benötigte Extraktionszeit beträgt vorzugsweise höchstens 16 h, bevorzugt höchstens 8 h und besonders bevorzugt höchstens 4 h. Die Wahl der gewählten Extraktionszeit erfolgt in Abhängigkeit von der Qualität des zu extrahierenden Holzes und von den übrigen Extraktionsbedingungen, dabei insbesondere von der Extraktionstemperatur.

Zusätzlich ist es besonders bevorzugt, die Extraktion in Schritt (3) unter Erwärmen und/oder unter Rückfluss des Extraktionsmittels durchzuführen. Die Extraktionstemperatur wird in Abhängigkeit vom verwendeten Extraktionsmittel eingestellt.

Die Extraktion erfolgt im Regelfall vorzugsweise bei Umgebungsdruck, kann aber selbstverständlich auch bei einem Unter- oder Überdruck durchgeführt werden. Vorzugsweise erfolgt die Extraktion bei einem Druck im Bereich von 800 bis 1200 mbar, bevorzugt bei einem Druck im Bereich von 900 bis 1100 mbar.

Die Extraktion wird vorzugsweise bei einer Temperatur im Bereich von 40 bis 120°C durchgeführt, vorzugsweise im Bereich von 50 bis 100°C.

Bei Verwendung eines wasser- oder ethanolhaltigen Extraktionsmittels, insbesondere eines Exraktionsmittelgemisches umfassend oder bestehend aus Wasser und Ethanol, ist eine Extraktionstemperatur von 50 - 100°C bevorzugt, vorzugsweise bei einem Druck im Bereich von 800 bis 1200 mbar, bevorzugt bei einem Druck im Bereich von 900 bis 1100 mbar, weiter bevorzugt bei einem Druck im Bereich von 950 bis 1050 mbar.

Der erfindungsgemäße oder erfindungsgemäß einzusetzende Holzextrakt sollte für die Verwendung in kosmetischen, dermatologischen oder pharmazeutischen Zubereitungen weitgehend frei oder gänzlich frei von in Schritt

(2) eingesetzten organischen Extraktionsmitteln sein. Das oder die organischen Extraktionsmittel und gegebenenfalls Wasser können in Schritt (4) durch ein geeignetes Verfahren (z.B. Destillation, Trocknung, Gefriertrocknung und ähnliche Verfahren) möglichst vollständig entfernt werden.

Daher umfasst ein Verfahren zur Herstellung eines erfindungsgemäßen bzw. erfindungsgemäß einzusetzenden Holzextraktes den Schritt (4)

partielles oder vollständiges Entfernen des oder der in Schritt (2) verwendeten organischen Extraktionsmittel, vorzugsweise vollständiges Entfernen des oder der in Schritt (2) verwendeten organischen Extraktionsmittel, bevorzugt vollständiges Entfernen des oder der in Schritt (2) verwendeten organischen Extraktionsmittel und Wasser.

Bevorzugt ist oder sind die Extraktionsmittel des Schrittes (2) ausgewählt aus der Gruppe bestehend aus Wasser, Methanol, Ethanol, n-Propanol, Isopropanol und Mischungen zweier oder mehrerer dieser Extraktionsmittel, da für die mit diesen Extraktionsmitteln erhältlichen *Gleditsia triacanthos* Holzextrakte eine höhere Aktivität im Sinne der vorliegenden Erfindung gefunden wurde.

Bevorzugte erfindungsgemäße Extrakte sind hergestellt oder herstellbar mit einem Verfahren umfassend oder bestehend aus den Schritten:

(1) Bereitstellen von Holzraspat, vorzugsweise von Kernholzraspat, der Art *Gleditsia triacanthos,*
(2) Versetzen des in Schritt (1) bereitgestellten Holzes mit einem Extraktionsmittel ausgewählt aus der Gruppe bestehend aus Wasser, Methanol, Ethanol, n-Propanol, Isopropanol und Mischungen zweier oder mehrerer dieser Extraktionsmittel,
(3) 1 bis zu 16 h Extrahieren des in Schritt (2) mit dem Extraktionsmittel versetzten Holzes zum Gewinnen eines Holzextraktes, und
(4) gegebenenfalls partielles oder vollständiges Entfernen des oder der in Schritt (2) verwendeten Extraktionsmittel.

Weiter bevorzugte erfindungsgemäße Extrakte sind hergestellt oder herstellbar mit einem Verfahren umfassend oder bestehend aus den Schritten:

(1) Bereitstellen von Kernholzraspat der Art *Gleditsia triacanthos,*

(2) Versetzen des in Schritt (1) bereitgestellten Holzes mit einem Extraktionsmittel ausgewählt aus der Gruppe bestehend aus Wasser, Ethanol und Mischungen von Wasser und Ethanol,

(3) 1 bis zu 8 h Extrahieren des in Schritt (2) mit dem Extraktionsmittel versetzten Holzes zum Gewinnen eines Holzextraktes, und

(4) vorzugsweise partielles oder vollständiges Entfernen des oder der in Schritt (2) verwendeten organischen Extraktionsmittel.

Weiter bevorzugte erfindungsgemäße Extrakte sind hergestellt oder herstellbar mit einem Verfahren umfassend oder bestehend aus den Schritten:

(1) Bereitstellen von Kernholzraspat der Art *Gleditsia triacanthos,*

(2) Versetzen des in Schritt (1) bereitgestellten Holzes mit einem Extraktionsmittel, das zumindest 80 Gew.-%, vorzugsweise zumindest 90 Gew.-%, bevorzugt zumindest 95 Gew.-% Wasser und Ethanol umfasst oder daraus besteht,

(3) 1 bis zu 8 h, vorzugsweise 1 bis 4 h, Extrahieren des in Schritt (2) mit dem Extraktionsmittel versetzten Holzes zum Gewinnen eines Holzextraktes, und

(4) partielles oder vollständiges Entfernen des oder der in Schritt (2) verwendeten organischen Extraktionsmittel.

Wenn ein Extraktionsmittel Wasser und Ethanol umfasst oder aus Wasser und Ethanol besteht, liegt das Gewichtsverhältnis von Wasser und Ethanol in Schritt (2) vorzugsweise im Bereich von 3 : 1 bis 1 : 3, bevorzugt im Bereich von 2 : 1 bis 1 : 2, besonders bevorzugt im Bereich von 3 : 2 bis 2 : 3, weiter bevorzugt im Bereich von 5 : 4 bis 4 : 5, am meisten bevorzugt bei 1 : 1.

Die erfindungsgemäßen Extrakte sind keine Extrakte, wie sie durch Extraktion des Kernholzes von *Gleditsia*

*triacanthos* mit Diethylether erhalten werden.

Bevorzugt sind die erfindungsgemäßen Extrakte keine Extrakte, wie sie durch Extraktion des Kernholzes von *Gleditsia triacanthos* mit Ether erhalten werden.

Der oben beschriebene erfindungsgemäße bzw. erfindungsgemäß einzusetzende *Gleditsia* Holzextrakt kann vorzugsweise weiterverarbeitet werden zu einem erfindungsgemäßen *Gleditsia* Holzextrakt in Feststoffform, indem das erfindungsgemäße Herstellverfahren ergänzt wird um die Schritte:

(5) Versetzen des durch ein geeignetes Verfahren (z.B. Destillation) aufkonzentrierten flüssigen Extrakts mit einem pharmazeutisch und/oder kosmetisch akzeptablen festen Träger, und

(6) Trocknen des mit dem Träger versetzten Extrakts durch ein geeignetes Verfahren (z.B. Sprüh- oder Bandtrockung)

Dabei kann Schritt (5) erfindungsgemäß auch entfallen, in diesem Fall wird ein höher konzentriertes Pulver als bei Zusetzen eines pharmazeutisch und/oder kosmetisch akzeptablen Trägers erhalten. Pharmazeutisch bzw. kosmetisch akzeptabel ist dabei ein solcher Feststoff, der für den Organismus, an dem er verwendet werden soll, zumindest nicht toxisch ist. Ein bevorzugter kosmetisch akzeptabler Feststoff ist pulverförmiges Maltodextrin.

Auf diese Weise lassen sich gut haltbare erfindungsgemäße Extrakte herstellen. Durch das Einstellen des Mischungsverhältnisses des erhaltenen Extrakts und des pharmazeutisch und/oder kosmetisch akzeptablen Trägers kann zudem auf vorteilhaft leichte Art die Endkonzentration der im Extrakt-Pulver enthaltenen Wirkstoffe eingestellt werden.

Weiterhin können der erfindungsgemäße bzw. erfindungsgemäß einzusetzende *Gleditsia* Holzextrakt bzw. eine *Gleditsia* Holzextrakt-haltige feste oder flüssige Zubereitung auch erfindungsgemäß durch Verkapselung weiterverarbeitet werden. Wobei der Extrakt bzw. die feste oder flüssige Zubereitung verkapselt wird mit einem festen Hüllmaterial, das vorzugsweise ausgewählt ist aus Stärken, abgebaute oder chemisch oder physikalisch modifizierte Stärken (insbesondere Dextrine und Maltodextrine), Gelatinen, Gummi Arabicum, Agar-Agar, Ghatti-Gummi, Gellan Gum, modifizierte und nicht modifizierte Cellulosen, Pullulan, Curdlan, Carrageenane, Alginsäure, Alginate, Pektin, Inulin, Xanthan Gum und Mischungen zweier oder mehrerer der genannten Substanzen.

Darüber hinaus kann der erfindungsgemäße bzw. erfindungsgemäß einzusetzende *Gleditsia* Holzextrakt auch zu einer erfindungsgemäßen flüssigen Mischung bzw. Zubereitung weiterverarbeitet werden, indem der erfindungsgemäße bzw. erfindungsgemäß einzusetzende *Gleditsia* Holzextrakt gemischt wird mit einem flüssigen Verdünnungsmittel, vorzugsweise ausgewählt aus der Gruppe bestehend aus 1,2-Propandiol, 1,3-Propandiol, 1,2-Butandiol, 1,3-Butandiol, 1,4-Butandiol, Glycerin, 1,2-Pentandiol, Neutralölen, Mineralölen, Silikonöl, pflanzlichen Ölen, Fettalkoholen, Fettsäureestern, Ethanol, Wasser und Mischungen zweier oder mehrerer der genannten Verdünnungsmittel.

Solche verdünnten flüssigen erfindungsgemäßen bzw. erfindungsgemäß einzusetzenden *Gleditsia* Holzextrakte sind insbesondere für kosmetische, dermatologische und pharmazeutische Zwecke gut weiterverarbeitbar. Gegebenenfalls können diese erfindungsgemäßen Mischungen bzw. Zubereitungen hergestellt werden unter Zusatz eines Konservierungsmittels, eines Lösungsvermittlers, eines Antioxidans und/oder Stabilisators.

Die Erfindung betrifft ferner eine Mischung enthaltend

(a) einen Holzextrakt
hergestellt oder herstellbar mit einem Verfahren umfassend oder bestehend aus den Schritten:

(1) Bereitstellen von Holz, vorzugsweise von Holzraspat, von *Gleditsia triacanthos,*

(2) Versetzen des in Schritt (1) bereitgestellten Holzes mit einem Extraktionsmittel ausgewählt aus der Gruppe bestehend aus Wasser, Methanol, Ethanol, n-Propanol, Isopropanol, Aceton, Essigester, überkritischem Kohlendioxid und Mischungen zweier oder mehrerer dieser Extraktionsmittel,

(3) bis zu 24 h Extrahieren des in Schritt (2) mit dem Extraktionsmittel versetzten Holzes zum Gewinnen eines Holzextraktes,
und

(4) gegebenenfalls partielles oder vollständiges Entfernen des oder der in Schritt (2) verwendeten Extraktionsmittel,
und

(b) ein oder mehrere mehrwertige Alkohole mit 3 oder 4 C-Atomen, vorzugsweise ausgewählt aus der Gruppe

bestehend aus 1,2-Propandiol, 1,3-Propandiol, 1,2-Butandiol, 1,3-Butandiol, 1,4-Butandiol und Glycerin, sowie gegebenenfalls zusätzlich

(c) Wasser
und/oder

(d) Ethanol
und

(e) einem oder mehreren Konservierungsmitteln gewählt aus der Gruppe bestehend aus Benzoesäure, ihre Ester und Salze, Propionsäure und ihre Salze, Salicylsäure und ihre Salze, 2,4-Hexadiensaeure (Sorbinsäure) und ihre Salze, Ester der p-Hydroxybenzoesäure (Parabene), Formaldehyd und Paraformaldehyd, 2-Hydroxybiphenylether und seine Salze, 2-Zinksulfidopyridin-N-oxid, anorganische Sulfite und Bisulfite, Natriumiodat, Chlorbutanolum, 4-Ethylquecksilber-(II)5-Amino-1,3-bis(2-Hydroxybenzoesaeure, ihre Salze und Ester, Dehydracetsäure, Ameisensäure, 1,6-Bis(4-amidino-2-bromphenoxy)-n-hexan und seine Salze, das Natriumsalz der Ethylquecksilber-(II)-thiosalicylsäure, Phenylquecksilber und seine Salze, 10-Undecylensaeure und ihre Salze, 5-Amino-1,3-bis(2-ethylhexyl)-5-methyl-hexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxydiphenylether, 4-Chlor-3,5-dimethylphenol, 1,1'-Methylen-bis(3-(1-hydroxymethyl-2,4-dioximidazolidin-5-yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1-(4-Chlorphenoxy)1(1H-imidazol-1-yl)-3,3-dimethyl-2-butanon, 1,3-Bis-(hydroxymethyl)-5,5-dimethyl-2,4-imidazolidindion, Benzylalkohol, Octopirox, 1,2-Dibrom-2,4-dicyanobutan, 2,2'-Methylenbis(6-brom-4-chlorphenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxypropan-2-ol, N-Alkyl($C_{12}$-$C_{22}$)trimethyl-ammoniumbromid und -chlorid, 4,4-Dimethyl-1,3-oxazolidin, N-Hydroxymethyl-N-(1,3-di(hydroxymethyl)-2,5-dioxoimidazolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-($C_8$-$C_{18}$)-dimethyl-benzyl-ammoniumchlorid, Alkyl-($C_8$-$C_{18}$)-dimethyl-benzylammonium-bromid, Alkyl-($C_8$-$C_{18}$)-dimethyl-benzyl-ammoniumsaccharinat, Benzylhemiformal, 3-Iod-2-propinyl-butylcarbamat, Natriumhydroxymethyl-aminoacetat oder Natriumhydroxymethylaminoacetat,

wobei die Gesamtmenge an Konservierungsmittel vorzugsweise im Bereich von 0.05 bis 5 Gew.% bezogen auf das Gesamtgewicht der Mischung ist.

Solche Mischungen sind besonders geeignet zur Einarbeitung in kosmetische, dermatologische oder pharmazeutische Zubereitungen, insbesondere in Zubereitungen in Form von Emulsionen.

Vorzugsweise beträgt die Gesamtmenge an Bestandteil (b) einer erfindungsgemäßen Mischung zumindest 10 Gew.-%, bevorzugt 20 bis 80 Gew.-%, weiter bevorzugt 35 bis 70 Gew.-%, jeweils bezogen auf das Gesamtgewicht der Mischung.

Vorzugsweise beträgt die Gesamtmenge an Wasser einer erfindungsgemäßen Mischung zumindest 5 Gew.-%, bevorzugt 10 bis 70 Gew.-%, weiter bevorzugt 15 bis 60 Gew.-%, jeweils bezogen auf das Gesamtgewicht der Mischung.

Bevorzugte erfindungsgemäße Mischungen umfassen

(a) einen Kernholzextrakt,
hergestellt oder herstellbar mit einem Verfahren umfassend oder bestehend aus den Schritten:

(1) Bereitstellen von Holz, vorzugsweise von Holzraspat, von *Gleditsia triacanthos,*

(2) Versetzen des in Schritt (1) bereitgestellten Holzes mit einem Extraktionsmittel ausgewählt aus der Gruppe bestehend aus Wasser, Methanol, Ethanol, n-Propanol, Isopropanol, Aceton, Essigester, überkritischem Kohlendioxid und Mischungen zweier oder mehrerer dieser Extraktionsmittel,

(3) bis zu 24 h Extrahieren des in Schritt (2) mit dem Extraktionsmittel versetzten Holzes zum Gewinnen eines Holzextraktes,
und

(4) gegebenenfalls partielles oder vollständiges Entfernen des oder der in Schritt (2) verwendeten Extraktionsmittel,

(b) ein oder mehrere mehrwertige Alkohole mit 3 oder 4 C-Atomen, vorzugsweise ausgewählt aus der Gruppe bestehend aus 1,2-Propandiol, 1,3-Butandiol und Glycerin,
und
(c) Wasser.

Auch kann ein erfindungsgemäßer bzw. erfindungsgemäß einzusetzender *Gleditsia triacanthos* Holzextrakt in zahlreichen Fällen vorteilhaft in Kombination mit einem oder mehreren Konservierungsmitteln eingesetzt werden. Vorzugsweise gewählt werden hierbei Konservierungsmittel wie Benzoesäure, ihre Ester und Salze, Propionsäure und ihre Salze, Salicylsäure und ihre Salze, 2,4-Hexadiensäure (Sorbinsäure) und ihre Salze, Ester der p-Hydroxybenzoesäure (Parabene), Formaldehyd und Paraformaldehyd, 2-Hydroxybiphenylether und seine Salze, 2-Zinksulfidopyridin-N-oxid, anorganische Sulfite und Bisulfite, Natriumiodat, Chlorbutanolum, 4-Ethylquecksilber-(II)5-Amino-1,3-bis(2-Hydroxybenzoesäure, ihre Salze und Ester, Dehydracetsäure, Ameisensäure, 1,6-Bis(4-amidino-2-bromphenoxy)-n-hexan und seine Salze, das Natriumsalz der Ethylquecksilber-(II)-thiosalicylsäure, Phenylquecksilber und seine Salze, 10-Undecylensäure und ihre Salze, 5-Amino-1,3-bis(2-ethylhexyl)-5-methyl-hexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxydiphenylether, 4-Chlor-3,5-dimethylphenol, 1,1'-Methylen-bis(3-(1-hydroxymethyl-2,4-dioximidazolidin-5-yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1-(4-Chlorphenoxy)1(1H-imidazol-1-yl)-3,3-dimethyl-2-butanon, 1,3-Bis-(hydroxymethyl)-5,5-dimethyl-2,4-imidazolidindion, Benzylalkohol, Octopirox, 1,2-Dibrom-2,4-dicyanobutan, 2,2'-Methylen-bis(6-brom-4-chlorphenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxypropan-2-ol, N-Alkyl($C_{12}$-$C_{22}$)trimethyl-ammoniumbromid und -chlorid, 4,4-Dimethyl-1,3-oxazolidin, N-Hydroxymethyl-N-(1,3-di(hydroxymethyl)-2,5-dioxoimidazolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-($C_8$-$C_{18}$)-dimethyl-benzyl-ammoniumchlorid, Alkyl-($C_8$-$C_{18}$)-dimethyl-benzylammonium-bromid, Alkyl-($C_8$-$C_{18}$)-dimethyl-benzyl-ammoniumsaccharinat, Benzylhemiformal, 3-Iod-2-propinyl-butylcarbamat, Natrium-hydroxymethyl-aminoacetat oder Natriumhydroxymethylaminoacetat.

Die Gesamtmenge an Konservierungsmittel (ein oder mehrere Substanzen) liegt vorzugsweise im Bereich von 0,05 bis 5 Gew.-%, bevorzugt im Bereich von 0,1 bis 2 Gew.-%, weiter bevorzugt im Bereich von 0,2 bis 1,5 Gew.-%, besonders bevorzugt im Bereich von 0,3 bis 1 Gew.-%, jeweils bezogen auf das Gesamtgewicht der Mischung.

Bevorzugte Konservierungsmittel sind dabei ausgewählt aus der Gruppe bestehend aus Benzoesäure, ihren Estern und Salzen, Propionsäure und ihren Salzen, 2,4-Hexadiensäure (Sorbinsäure) und ihren Salzen, Estern der p-Hydroxybenzoesäure (Parabene), Benzylalkohol, 1-Phenoxypropan-2-ol und 2-Phenoxyethanol.

Weiter bevorzugte erfindungsgemäße Mischungen umfassen

(a) einen erfindungsgemäßen Kernholzextrakt von *Gleditsia triacanthos,*

(b) 1,2-Propandiol, 1,3-Butandiol und/oder Glycerin, vorzugsweise in einer Gesamtmenge von 20 bis 80 Gew.-%, bevorzugt 40 bis 70 Gew.-%, jeweils bezogen auf das Gesamtgewicht der Mischung,

(c) Wasser
und

ein oder mehrere Konservierungsmittel, vorzugsweise ausgewählt aus der Gruppe bestehend aus Benzoesäure und ihren Salzen, 2,4-Hexadiensäure (Sorbinsäure) und ihren Salzen, Estern der p-Hydroxybenzoesäure (Parabene) und 2-Phenoxyethanol, wobei die Gesamtmenge an Konservierungsmittel vorzugsweise im Bereich von 0,2 bis 1,5 Gew.-% liegt, bevorzugt im Bereich von 0,3 bis 1 Gew.-%, jeweils bezogen auf das Gesamtgewicht der Mischung.

Hierdurch erhalten die erfindungsgemäßen Mischungen besonders hohe Lagerstabilität.

In einer bevorzugten Ausgestaltung enthält eine erfindungsgemäße Mischung mindestens ein Konservierungsmittel, vorzugsweise mindestens zwei Konservierungsmittel aus der Gruppe Benzoesäure, Natriumbenzoat, Kaliumbenzoat, Sorbinsäure, Natriumsorbat und Kaliumsorbat.

In einer bevorzugten Ausgestaltung liegt pH-Wert einer erfindungsgemäßen Mischung vorzugsweise im Bereich von 3 bis 7, bevorzugt im Bereich von 4 bis 6, jeweils bei 25°C. Dabei wird der pH-Wert einer erfindungsgemäßen Mischung vorzugsweise durch Zugabe von Citronensäure und/oder Milchsäure eingestellt.

Erfindungsgemäße Zubereitungen enthaltend einen erfindungsgemäßen bzw. erfindungsgemäß einzusetzenden *Gleditsia triacanthos* Holzextrakt bzw. eine erfindungsgemäße Mischung beeinflussen die Cellulite im Hinblick auf die eingelagerte Lipidmenge, indem der Lipidgehalt im humanen Unterhaut-Fettgewebe reduziert wird. So wird Cellulite durch eine Zubereitung enthaltend einen erfindungsgemäßen bzw. erfindungsgemäß einzusetzenden *Gleditsia triacanthos* Holzextrakt insbesondere durch die positive Beeinflussung der oben beschriebenen Wege (i) (Adipogenese) und (ii) (Lipogenese) vorgebeugt, behandelt oder verringert.

Die erfindungsgemäßen bzw. erfindungsgemäß einzusetzenden *Gleditsia triacanthos* Holzextrakte zeigen eine ausgeprägte Wirkung bei der Behandlung von Cellulite, beispielsweise erkennbar mittels echographischer Bestimmung der Subcutis-Schichtdicke, insbesondere zur Vorbeugung der verstärkten Bildung von Fettdepots in der Haut und/oder Cellulite, indem der Lipidgehalt im humanen Unterhaut-Fettgewebe reduziert wird.

Die Erfindung betrifft daher auch Zubereitungen, vorzugsweise kosmetische Zubereitungen, enthaltend eine entsprechende wirksame Menge eines erfindungsgemäßen bzw. erfindungsgemäß einzusetzenden *Gleditsia tricanthos* Holzextraktes, insbesondere zur topischen Behandlung und Vorbeugung der verstärkten Bildung von Fettdepots in der Haut und/oder Cellulite.

Außerdem wirken die erfindungsgemäßen Zubereitungen positiv auf Hautreizungen, die mit einer Cellulite einhergehen können, da - wie oben bereits ausgeführt - die erfindungsgemäßen bzw. erfindungsgemäß einzusetzenden *Gleditsia triacanthos* Holzextrakte neben anti-cellulite - Wirksamkeit zusätzlich SIRT-stimulierende / -aktivierende und anti-irritierende Wirksamkeit aufweisen.

Ein weiterer Gegenstand der vorliegenden Erfindung ist eine nicht-therapeutische Verwendung einer kosmetischen, dermatologischen oder pharmazeutischen Zubereitung, enthaltend einen Holzextrakt von *Gleditsia triacanthos* zur Vorbeugung, Behandlung oder Verringerung von Cellulite.

Eine bevorzugte erfindungsgemäße Zubereitung enthält einen erfindungsgemäßen Holzextrakt, vorzugsweise in einer der oben als bevorzugt gekennzeichneten Ausgestaltungen, oder eine erfindungsgemäße Mischung, vorzugsweise in einer der oben als bevorzugt gekennzeichneten Ausgestaltungen.

Eine weiter bevorzugte erfindungsgemäße Zubereitung enthält einen erfindungsgemäßen Holzextrakt, vorzugsweise einen Kernholzextrakt, von *Gleditsia triacanthos,* vorzugsweise in einer der oben als bevorzugt gekennzeichneten Ausgestaltungen.

Es wurde ferner gefunden, dass ein erfindungsgemäßer bzw. erfindungsgemäß einzusetzender Holzextrakt eine höhere Aktivität im Sinne der vorliegenden Erfindung zeigen, wenn der *Gleditsia triacanthos* Holzextrakt Fustin (3,3',4',7-Tetrahydroxyflavanon) enthält und vorzugsweise sowohl Fustin als auch Fisetin (3,3',4',7-Tetrahydroxyflavon) enthält.

Es hat sich darüberhinaus in weiteren eigenen Untersuchungen gezeigt, dass weder Fustin noch Fisetin in entsprechender Konzentration eine Aktivität bezüglich der Adipogenese zeigten (siehe Beispiel 2). Folglich sind diese beiden Verbindungen für die Adipogenese hemmende Wirkung eines erfindungsgemäßen bzw. erfindungsgemäß einzusetzenden Holzextraktes nicht oder zumindest nicht nennenswert verantwortlich.

Somit beruht die Wirksamkeit der erfindungsgemäßen bzw. erfindungsgemäß einzusetzenden Holzextrakte auf noch nicht identifizierten bzw. charakterisierten Substanzen. Unter den oben angegebenen Extraktionsbedingungen, unter denen Fustin, vorzugsweise Fustin und Fisetin, aus dem *Gleditsia triacanthos* Holz extrahiert werden, werden auch die noch nicht identifizierten bzw. charakterisierten Wirkstoffe extrahiert, die für die Wirksamkeit im Sinne der vorliegenden Erfindung verantwortlich sind. Ein bevorzugter erfindungsgemäßer bzw. erfindungsgemäß einzusetzender *Gleditsia triacanthos* Holzextrakt enthält daher Fustin, vorzugsweise Fustin und Fisetin. Entsprechend enthält eine bevorzugte erfindungsgemäße Mischung bzw. eine bevorzugte erfindungsgemäße Zubereitung einen erfindungsgemäßen bzw. erfindungsgemäß einzusetzenden Holzextrakt, der Fustin und vorzugsweise zusätzlich Fisetin enthält.

Ein bevorzugter erfindungsgemäßer bzw. erfindungsgemäß einzusetzender *Gleditsia triacanthos* Holzextrakt enthält neben Fustin auch Protocatechusäure (3,4-Dihydroxybenzoesäure), vorzugsweise Fustin, Fisetin und Protocatechusäure.

In einer bevorzugten Ausgestaltung ist ein erfindungsgemäßer bzw. erfindungsgemäß einzusetzender Holzextrakt, eine erfindungsgemäße Mischung bzw. eine erfindungsgemäße Zubereitung frei von Gledistin.

Wesentliche Anwendungsgebiete sind hierbei kosmetische, insbesondere dermatologische Zubereitungen, die (abgesehen von dem erfindungsgemäßen Extrakt) wie üblich zusammengesetzt sind und dem kosmetischen, insbesondere dermatologischen, Lichtschutz, zur Behandlung, der Pflege und der Reinigung der Haut und/oder der Haare oder als Schminkprodukt in der dekorativen Kosmetik dienen. Entsprechend können derartige Zubereitungen, je nach ihrem Aufbau beispielsweise als Hautschutzcreme, Tages- oder Nachtcreme, Augencreme, Sonnenschutz- oder Aftersun-Lotion, Nährcreme, Pflegemaske, Gel-Pads, Gesichtswasser, feuchte Pflege- und Reinigungstücher, Reinigungsmilch, Reinigungsseife, Schaum- oder Duschbad, Deodorant, Antitranspirant, Haarshampoo, Haarpflegemittel, Haarconditioner, Haarcoloration, Haarstylingmittel verwendet werden und dabei bevorzugt als Emulsion, Lotion, Milch, Fluid, Creme, Hydrodispersionsgel, Balm, Spray, alkoholische oder wässrig/alkoholische Lösung, Schaum, Puder, Flüssigseife, Seifenstück, Shampoo, Roll-on, Stick oder Make-up vorliegen. In Haarbehandlungsmitteln richtet sich die Verwendung vorzugsweise auf den Haarboden oder die Kopfhaut.

Ein weiterer Aspekt der vorliegenden Erfindung bezieht sich auf Mundpflegeprodukte (Mundhygieneprodukte), wobei die Mundpflegeprodukte vorzugsweise in Form von Zahnpasta, Zahncreme, Zahngel, Zahnpulver, Zahnreinigungsflüssigkeit, Zahnreinigungsschaum, Mundwasser (gebrauchsfertig oder als Konzentrat), Zahncreme und Mundwasser als 2-in-1 Produkt, zuckerfreies Lutschbonbon, Mundspray, Zahnseide oder Zahnpflegekaugummi vorliegen.

Die Menge eines erfindungsgemäßen bzw. erfindungsgemäß einzusetzenden *Gleditsia triacanthos* Holzextraktes, in einer erfindungsgemäßen kosmetischen, dermatologischen oder pharmazeutischen Zubereitung liegt, bezogen auf die Masse des Trockenextraktes (wie oben definiert), vorzugsweise im Bereich von 0,00001 bis 10 Gew.-%, bevorzugt im Bereich von 0,0001 bis 5 Gew.-%, weiter bevorzugt im Bereich von 0,001 bis 2 Gew.-%, besonders bevorzugt im Bereich von 0,001 bis 1 Gew.-%, ganz besonders bevorzugt im Bereich von 0,002 bis 0,5 Gew.-% und am meisten bevorzugt im Bereich von 0,002 bis 0,2 Gew.-%, jeweils bezogen auf die Gesamtmasse der kosmetischen, dermatologischen bzw. pharmazeutischen Zubereitung.

Die erfindungsgemäßen bzw. erfindungsgemäß einzusetzenden *Gleditsia triacanthos* Holzextrakte bzw. eine erfindungsgemäße *Gleditsia tricanthos* Holzextrakt-haltige festen oder flüssigen Formulierungen bzw. Mischungen lassen sich ohne Schwierigkeiten in gängige kosmetische oder dermatologische Formulierungen wie Pumpsprays, Aerosolsprays, Cremes, Salben, Tinkturen, Lotionen, Nagelpflegeprodukte und dergleichen einarbeiten. Diese *Gleditsia triacanthos* Holzextrakt enthaltenden kosmetischen und/oder dermatologischen Formulierungen können hierbei ansonsten wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung in der für Kosmetika und Dermatika üblichen Weise werden die erfindungsgemäßen Zubereitungen auf die Haut und/oder die Haare in ausreichender Menge aufgebracht.

In einem weiteren Aspekt betrifft die Erfindung eine dermatologische oder pharmazeutische Zubereitung, enthaltend einen Holzextrakt von *Gleditsia triacanthos* in einer ausreichenden Menge zur Verwendung als Medikament zum

- Hemmen der Differenzierung von Preadipozyten,
  und/oder
- Hemmen der Lipogenese in Adipozyten,
  und/oder
- Reduzieren der in subkutanem Fettgewebe enthaltenen Lipidmenge,

Bevorzugt enthält eine Zubereitung in einer erfindungsgemäßen nicht-therapeutischen Verwendung oder erfindungsgemäßen dermatologischen oder pharmazeutischen Zubereitung, eine Mischung, enthaltend einen erfindungsgemäßen Holzextrakt von *Gleditsia triacanthos* Hierbei ist es auch möglich und regelmäßig vorteilhaft, den erfindungsgemäßen bzw. erfindungsgemäß einzusetzenden Holzextrakt mit weiteren Wirkstoffen zu kombinieren.

Substanzen und Hilfsstoffe, welche eine erfindungsgemäße kosmetische, dermatologische und/oder pharmazeutische Zubereitung enthaltend einen erfindungsgemäßen Extrakt, zusätzlich enthalten kann, sind beispielsweise:

Konservierungsmittel, vorzugsweise die in US 2006/0089413 genannten, Abrasiva, Antiakne-Mittel und Mittel zu Sebumreduktion, vorzugsweise die in WO 2008/046791 genannten, weitere Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, weitere Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-beta-dicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, $\alpha$-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen und Geschmackstoffe sowie Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, haarwachstumsmodulierende Mittel (haarwachstumsfördend oder haarwachstumshemmend), vorzugsweise die in EP 2168570 und EP 2193785 genannten oder Silikone und Silikonderivate, vorzugsweise die in WO 2008/046676 genannten.

Eine bevorzugte erfindungsgemäße Zubereitung umfasst neben einem erfindungsgemäßen bzw. erfindungsgemäß einzusetzenden Holzextrakt ferner ein, zwei, drei oder mehrere Stoffe ausgewählt aus der Gruppe bestehend aus:

- Lipolyse-Stimulantien, vorzugsweise ausgewählt aus

  (b-i) der Gruppe der Phosphodiesterase-Inhibitoren, und/oder
  (b-ii) der Gruppe der Agonisten beta-adrenerger Rezeptoren,

- der Gruppe der weiteren Sirtuin-Aktivatoren,
  und
- der Gruppe der UV-Strahlung absorbierenden Mittel.

Insbesondere Zubereitungen enthaltend einen erfindungsgemäßen bzw. erfindungsgemäß einzusetzenden *Gleditsia triacanthos* Holzextrakt und ein oder mehrere Lipolyse-Stimulantien, vorzugsweise ausgewählt aus der Gruppe der Phosphodiesterase-Inhibitoren, und/oder der Gruppe der Agonisten beta-adrenerger Rezeptoren, erreichen eine besonders gute anti-cellulite Wirksamkeit. Cellulite wird durch eine solche Zubereitung durch die positive Beeinflussung der oben beschriebenen Wege (i) bis (iii) besonders effektiv vorgebeugt, behandelt oder verringert: insbesondere die Wege (i) (Adipogenese) und (ii) (Lipogenese) werden durch einen erfindungsgemäßen bzw. erfindungsgemäß einzusetzenden Holzextrakt verstärkt positiv beeinflusst und Weg (iii) durch weitere Lipolyse-Stimulantien.

In einer bevorzugten Ausgestaltung enthält eine erfindungsgemäße Zubereitung, insbesondere zur nicht-therapeutischen Anwendung als anti-cellulite Mittel, zusätzlich ein, zwei drei oder mehrere Lipolyse-Stimulantien umfasst, wobei einer mehrere oder alle der Lipolyse-Stimulantien

- eine Verbindung gewählt aus der Gruppe der gegebenenfalls substituierten 3,7- oder 3,9-Dihydro-1*H*-purin-2,6-dione der Formel (Xa) oder ein pharmazeutisch akzeptables Salz einer solchen Verbindung ist,

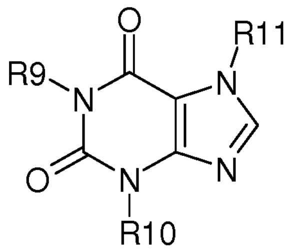

(Xa)

wobei R9, R10 und R11 unabhängig voneinander Wasserstoff oder Methyl bedeuten und/oder

- eine Verbindung der Formel (PhEA) oder ein pharmazeutisch akzeptables Salz einer solchen Verbindung ist,.

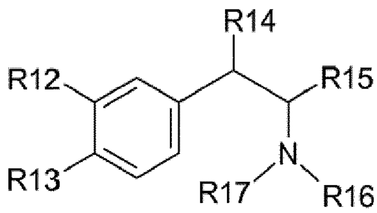

(PhEA)

wobei, unabhängig voneinander,

R12 und R13 jeweils Wasserstoff, Hydroxy und Methoxy,
R14 Wasserstoff, Hydroxy und Methyl,
R15 Wasserstoff und Methyl und
R16 und R17 jeweils Wasserstoff und C1-C4-Alkyl bedeuten.

Die Xanthine, insbesondere die der Formel (Xa), können vorzugsweise als Reinstoffe oder auch in Form von Pflanzenextrakten eingesetzt werden.

Bevorzugt sind die Methylxanthine Coffein (R9 = R10 = R11 = $CH_3$), Theobromin (R9 = H, R10 = R11 = $CH_3$) und Theophyllin (R9 = R10 = $CH_3$, R11 = H), das am meisten im Sinne der vorliegenden Erfindung bevorzugte Xanthin ist Coffein. Ebenfalls bevorzugt ist das Theophyllin-Derivat Aminophyllin.

Besonders bevorzugt enthält eine kosmetische, vorzugsweise topische, erfindungsgemäße Zubereitung eine oder mehrere Verbindungen der Formel (Xa), dabei wiederum bevorzugt Coffein. Die Gesamtmenge an Verbindungen der Formel (Xa) beträgt vorzugsweise 0,005 - 10 Gew.-%, bevorzugt 0,05 - 5 Gew.-%, besonders bevorzugt 0,5 - 2,5 Gew.-%, jeweils bezogen auf das Gesamtgewicht der Zubereitung, wobei eventuelle Gegenionen der Verbindungen der Formel (Xa) nicht mitgerechnet werden.

Ebenfalls bevorzugte Zubereitungen enthalten Kombinationen von erfindungsgemäßem bzw. erfindungsgemäß einzusetzendem *Gleditsia triacanthos* Holzextrakt mit einem Agonisten beta-adrenerger-Rezeptoren der Adipozyten.

Bevorzugte Agonisten beta-adrenerger-Rezeptoren sind β-Phenylethylamine der Formel (PhEA) oder ein pharmazeutisch akzeptables Salz einer Verbindung der Formel (PhEA)

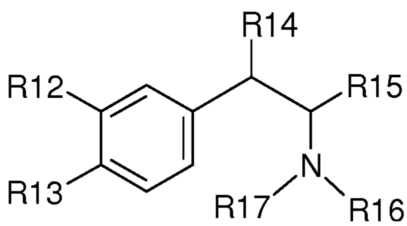

(PhEA)

wobei

R12 und R13, unabhängig voneinander, Wasserstoff, Hydroxy oder Methoxy,
R14 Wasserstoff, Hydroxy oder Methyl,
R15 Wasserstoff oder Methyl,
R16 und R17, unabhängig voneinander, Wasserstoff oder $C_1$-$C_4$-Alkyl,

bedeuten.

Die β-Phenylethylamine der Formel (PhEA) können vorzugsweise als Reinstoffe, in Form ihrer jeweiligen Hydrochloride oder in Form von Pflanzenextrakten eingesetzt werden. Bevorzugte Agonisten beta-adrenerger-Rezeptoren sind Adrenalin, Noradrenalin, Metanephrin, Macromerin, Normacromerin, Hordenin, N-Methyltyramin, Dopamin, Octopamin, Tyramin, 2-Phenylethylamin, Phenylethanolamin, Epinin (N-Methyldopamin), Synephrin, Ephedrin, Pseudoephedrin, Norephedrin und Isoprenalin.

Einige dieser Verbindungen wurden in der Literatur bereits auf ihre Aktivität hinsichtlich des beta-3-adrenergen-Rezeptors bei menschlichen Fettzellen sowie von Säugetieren untersucht (Naunyn-Schmiedeberg's Archives of Pharmacology 1999, 359, 310-321).

Bevorzugt sind Verbindungen der Formel (PhEA), bei denen R 17 = H ist und R16 Wasserstoff oder $C_1$-$C_4$-Alkyl, bevorzugt Wasserstoff, Methyl oder iso-Propyl, bedeutet. Ferner bevorzugt sind Verbindungen der Formel (PhEA), bei denen zusätzlich R15 = H ist.

In einer weiteren bevorzugten Ausgestaltung sind die Agonisten beta-adrenerger-Rezeptoren solche Verbindungen, bei denen R12 und R 17 = H ist.

Insbesondere bevorzugte Agonisten beta-adrenerger-Rezeptoren entsprechen der Formel (PhEA-i), oder einem pharmazeutisch akzeptablen Salz einer solchen Verbindung, dabei wiederum bevorzugt sind Tyramin, N-Methyltyramin, Octopamin, und Synephrin und deren pharmazeutisch akzeptablen Salze

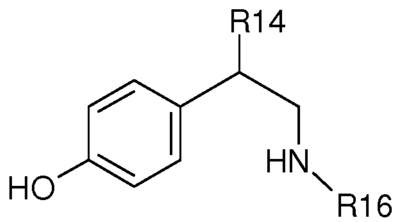

(PhEA-i)

wobei die Reste R14 und R16 die oben genannte (bevorzugte) Bedeutung haben.

Der am meisten bevorzugte Agonist eines beta-adrenergen-Rezeptors ist Synephrin (R14 = OH, R16 = $CH_3$ in Formel (PhEA-i)), vorzugsweise racemisch oder enantiomerenrein, dabei wiederum bevorzugt ist die (-)-Form. Ebenfalls besonders bevorzugt sind Synephrin-haltige Extrakte, wie beispielsweise Orangenblüten-Extrakt.

Bevorzugt enthält eine kosmetische, vorzugsweise topische, erfindungsgemäße Zubereitung einen Agonisten eines beta-adrenergen-Rezeptors, dabei bevorzugt Synephrin, vorzugsweise in einer Gesamtmenge von 0,0001 - 0,10 Gew.-%, bevorzugt von 0,001 - 0,05 Gew.-%, weiter bevorzugt von 0,002 - 0,02 Gew.-%, jeweils bezogen auf das Gesamtgewicht der Zubereitung, wobei bei Salzen das Gegenion des Agonisten nicht mitgerechnet wird.

Besondere Vorteile bieten kosmetische, dermatologische und pharamzeutische Zubereitungen, die einen erfindungsgemäßen Extrakt enthalten und einen, zwei, drei oder mehrere weitere Sirtuin-Aktivatoren. Vorteilhafte Sirtuin-Aktivatoren sind beispielsweise Resveratrol, Resveratrol Derivative, insbesondere Hydroxyresveratrol und Resveratrolphosphate wie Resveratroltriphosphat, Butein, Piceatannol, Isoliquiritigenin, Quercetin, Deoxyrhapontin, Rhapontin, Trihydroxychalkon, Pentahydroxychalkon, Chalkon, Tetrahydroxyflavone, Dihydroxyflavone, Kaempferol, Hydroxyapigenin, Apigenin, Myrecitin, Gossypetin, Morin, Daidzein, Genistein, Naringenin, Catechin, Epicatechin, Gallocatechin, Epigallocatechin, Hinokitiol, Ergothioneine, Ambroxol, Trolox, Dipyridamole, Camptothecin, Coumestrol, Pinosylvin, SIRT1-Aktivatoren wie beschrieben in US 2005/0136537, Isonicotinamid, epsilon-Viniferin, Prosveltyl (Silab) enthaltend Nelumbo nucifera Blatt Extrakt, Longevicell (Silab) enthaltend hadrolysierten Myrtus communis Blatt Extrakt, Sepivinol (Seppic) enthaltend Polyphenole aus Wein, Orsirtine (ISP Vincience) enthaltend Oryza Sativa (Reis) Extrakt, Peptides, insbesondere Kluyveromyces Peptide, Dynachondrine ISR (ISP Vincience) enthaltend hydrolysiertes Sojaprotein, Sirpetide (ISP Vincience) enthaltend Heptapeptide-6, ein synthetisches Peptid enthaltend Asparagin, Asparaginsäure, Glutaminsäure, Glycin, Leucin und Tyrosin, Matrispondin (Sederma SAS) enthaltend Palmitoyltetrapeptid-10 (ein synthetisches Peptid bestehend aus Lysin, Threonin und Phenylalanin), ACB Sirtuin Complex (Active Concepts) enthaltend Saccharomyces/Podophyllum peltatum Fermentfiltrat, Silibinin, Mariendiestel-Extrakte enthaltend Silibinin, Trigonella

foenum graecum Extrakt, Hefe-Extrakt, Echinacea purpurea Extrakt oder Presssaft, Dikaffeesäure-Derivate wie beispielsweise Cichorinsäure, Isochlorogensäuren (z.B. 4,5-Dicaffeoylchinasäure) sowie Dikaffeesäure-Derivat haltige Extrakte und Hydrangea arborescens Wurzel Extrakt.

Da sowohl die Hautalterung als auch das Auftreten von Cellulite auch mit einem Abbau des Bindegewebes einher geht, können erfindungsgemäß bevorzugte Zubereitungen enthaltend einen erfindungsgemäßen bzw. erfindungsgemäß einzusetzenden *Gleditsia* Holzextrakt auch Wirkstoffe enthalten, die einem Abbau des Bindegewebes vorbeugen. Vorteilhaft sind dabei Wirkstoffe, die Matrix-Metalloproteinasen hemmen. Diese Enzyme sind in der Lage, Makromoleküle der extrazellulären Matrix (ECM) / des Bindegewebes, zu der auch die Collagene zählen, proteolytisch abzubauen. Insbesondere die Matrix-Metalloproteinase-1 (MMP-1), Matrix-Metalloproteinase-2 (MMP-2) und Matrix-Metalloproteinase--9 (MMP-9) sind für den Abbau des Bindegewebes der Haut verantwortlich. Eine Inhibition von MMPs ist zum Beispiel durch den Zusatz von Ursolsäure, Retinylpalmitat, Propylgallat, Precocene, 6-Hydroxy-7-methoxy-2,2-dimethyl-1(2H)-benzopyran, 3,4-Dihydro-6-hydroxy-7-methoxy-2,2-dimethyl-1(2H)-benzopyran möglich. Auch ein Zusatz von Peptiden, die MMPs hemmen, zu erfindungsgemäßen Zubereitungen ist zur Hemmung von MMPs vorteilhaft. Auch Proteine oder Glycoproteine aus Soja sowie hydrolysierte Proteinen aus Reis, Erbse oder Lupine inhibieren MMPs und sind somit ein geeigneter Zusatz. Eine Kombination mit einem Pflanzenextrakt, der MMPs hemmt, ist ebenfalls von Vorteil. Zu nennen ist dabei beispielhaft ein Extrakt aus Shiitake-Pilzen. Vorteilhaft ist auch die Kombination mit Extrakten aus den Blättern der Familie Rosaceae, Unterfamilie Rosoideae. Ganz besonders vorteilhaft ist die Verwendung von Brombeerblatt-Extrakt, insbesondere wie beschrieben in WO 2005/123101 A1.

Im Rahmen der vorliegenden Erfindung bevorzugt in Kombination zu verwendende MMP-Inhibitoren sind Retinylpalmitat, Propylgallat, Precocene, 6-Hydroxy-7-methoxy-2,2-dimethyl-1 (2H)-benzopyran, 3,4-Dihydro-6-hydroxy-7-methoxy-2,2-dimethyl-1 (2H)-benzopyran, Benzamidin Hydroclorid, die Cysteinproteinase-Inhibitoren N-Ethylmaleimid und epsilon-Amino-n-Capronsäure, der Serinprotease-Inhibitor: Phenylmethylsulfonylfluorid, Collhibin (Fa. Pentapharm; INCI: Hydrolyzed Rice Protein), Oenotherol (Fa. Soliance; INCI: Propylene Glycol, Aqua, Oenothera biennis root extract, Ellagsäure und Ellagitannine, z.B. aus Granatapfel), Phosphoramidon Hinokitiol, EDTA, Galardin, EquiStat (Fa. Collaborative Group; Apfelfruchtextrakt, Sojasamenextrakt: Ursolsäure, Sojaisoflavone & Sojaproteine), Salbeiextrakte, MDI (Fa. Atrium; INCI: Glycosaminoglycans), Fermiskin (Fa. Silab/Mawi; INCI: Water and Lentinus Edodes Extract), Actimp 1.9.3. (Fa. Expanscience/ Rahn; INCI: Hydrolyzed Lupine Protein), Lipobelle Soyaglycone (Fa. Mibelle; INCI: Alcohol, Polysorbate 80, Lecithin and Soy Isoflavones), Extrakte aus grünem und schwarzem Tee sowie zahlreiche weitere Pflanzenextrakte, die in WO 02/069992 (siehe dort Tabellen 1-12) gelistet sind.

Um dem Abbau des Bindegewebes entgegenzuwirken, ist des Weiteren in erfindungsgemäß bevorzugten kosmetischen Zubereitungen enthaltend einen erfindungsgemäßen bzw. erfindungsgemäß einzusetzenden *Gleditsia* Holzextrakt die Kombination mit Wirkstoffen vorteilhaft, die die Bildung von Collagen im Gewebe fördern. Häufig zur Steigerung der Collagensynthese eingesetzte Einzelstoffe sind zum Beispiel Wirkstoffe wie Ascorbinsäure und deren Derivate, Retinol sowie Abkömmlinge des Retinols oder Pflanzenextrakte wie zum Beispiel Extrakte aus Aloe- und Centella-Arten. Darüber hinaus zählen auch peptidische Stoffe und deren Derivate wie z.B. Carnitin, Carnosin, Kreatin, Matrikine Peptide (e.g. lysyl-threonyl-threonyl-lysyl-serine) sowie weitere peptidische Strukturen wie palmitoylierte Pentapeptide (z.B. Matrixyl/Fa. Sederma) oder das Oligopeptid mit dem Handelsnamen Vincipeptide (Fa. Vincience/Frankreich) zu häufig verwendeten, die Collagensynthese steigernden Wirkstoffen. Im weiteren finden Verbindungen wie Asiatsäure (Asiatic acid), Madecassic Säure, Madecassosid, Asiaticosid, Extrakte aus Centella asiatica, Niacinamid, Astaxanthin, Glucane z.B aus Hefen und Hafer, Sojaextrakte sowie Sojaisoflavone wie Genistein und Daidzein, Rutin, Chrysin, Morin, Betelnuss-Alkaloide, Forskolin, Betulinsäure, Extrakte aus Plantago Arten, TGF-beta, Extrakte aus Ginkgo biloba, Glutamin und Glycolsäure Einsatz als Collagensynthesestimulatoren. Besonders bevorzugt ist dabei der Zusatz einer Kombination aus Aloe vera Extrakt, Himbeerblatt-Extrakt und Magnesiumascorbylphosphat.

Desweiteren bevorzugte erfindungsgemäße Zubereitungen enthaltend einen erfindungsgemäßen bzw. erfindungsgemäß einzusetzenden *Gleditsia triacanthos* Holzextrakt umfassen ferner zusätzlich (i) Coenzym A zur Förderung des Transportes der freien Fettsäuren in den Mitochondrien und/oder (ii) L-Carnitin zur Förderung der beta-Oxidation.

Daher betrifft die vorliegende Erfindung ferner eine Zubereitung, vorzugsweise in einer der als bevorzugt gekennzeichneten Ausgestaltungen, ferner enthaltend

- einen oder mehrere Matrix-Metalloproteinase-Hemmer, und/oder
- einen oder mehrere Collagensynthese-Stimulatoren, und/oder
- Coenzym A und/oder L-Carnitin.

Besondere Vorteile bieten ebenfalls, insbesondere kosmetische und dermatologische, erfindungsgemäße Zubereitungen, die einen erfindungsgemäßen Extrakt enthalten und zusätzlich als Sonnenschutzmittel wirken. Vorteilhaft enthalten diese Zubereitungen mindestens einen UVA-Filter und/oder mindestens einen UVB-Filter und/oder mindestens

ein anorganisches Pigment. Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol bilden.

Erfindungsgemäße Zubereitungen im Bereich Kosmetika und Dermatika, die einen erfindungsgemäßen Extrakt enthalten, werden besonders vorteilhaft mit Substanzen kombiniert, die UV-Strahlung absorbieren oder reflektieren, namentlich für kosmetische oder hautschützende Zwecke, wobei die Gesamtmenge der Filtersubstanzen von 0,01 Gew.-% bis 40 Gew.-%, vorzugsweise 0,1% bis 20 Gew.-%, insbesondere 1,0 bis 10 Gew.-% beträgt, bezogen auf das Gesamtgewicht der Zubereitungen, um kosmetische Zubereitungen zur Verfügung zu stellen, die das Haar bzw. die Haut vor ultravioletter Strahlung schützen. Vorteilhaft enthalten diese Zubereitungen mindestens einen UVA-Filter und/oder mindestens einen UVB-Filter und/oder mindestens ein anorganisches Pigment, so dass ein Lichtschutzfaktor (SPF) von zumindest > 2 (bevorzugt > 5) erreicht wird. Diese erfindungsgemäßen Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Vorteilhafte UV-Filter und anorganische Lichtschutzpigmente sind genannt in WO 2005/123101. Besonders zur Kombination geeignete UV-Absorber sind ebenfalls genannt in WO 2005/123101.

Vorteilhafte anorganische Lichtschutzpigmente sind feindisperse Metalloxide und Metallsalze die ebenfalls in WO 2005/123101 genannt sind. Die Gesamtmenge an anorganischen Pigmenten, insbesondere hydrophoben anorganischen Mikropigmenten in den fertigen kosmetischen oder dermatologischen Formulierungen liegt vorteilhaft im Bereich von 0,1 bis 30 Gew.-%, bevorzugt 0,1 bis 10 Gew.-%, insbesondere 0,5 bis 6 Gew.-%, bezogen auf das Gesamtgewicht der Formulierungen.

Weiterhin ist es vorteilhaft den erfindungsgemäßen Extrakt in kosmetischen oder dermatologischen Zubereitungen mit Aktivstoffen zu kombinieren, die in die Haut penetrieren und die Hautzellen von innen heraus gegen Sonnenlicht-induzierte schädigende Effekte wie Hautalterung, Hautentzündung und Hautkrebs schützen. Bevorzugte Wirkstoffe in diesem Zusammenhang sind sogenannte Arylhydrocarbon-Rezeptor-Antagonisten wie beschrieben in WO 2007/128723. Besonders bevorzugt ist 2-Benzylidene-5,6-dimethoxy-3,3-dimethylindan-1-on.

Besondere Vorteile bieten darüber hinaus kosmetische oder dermatologische Zubereitungen, die einen erfindungsgemäßen Extrakt enthalten und zusätzlich einen, zwei, drei oder mehrere Stimulatoren der Glycosaminoglycan (GAG)-Synthese. Vorteilhafte GAG-Synthese Stimulatoren sind beispielsweise Hyaluronsäure, SymVital (Symrise, INCI: Aloe Barbadensis leaf juice powder, Magnesium Ascorbyl Phosphate, Rubus Idaeus (Raspberry) leaf extract), Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), Glucosamin, N-Acetylglucosamin, Retinoide, bevorzugt Retinol und Vitamin A, Arctium lappa Frucht Extrakt, Eriobotrya japonica Extrakt, Genkwanin, N-Methyl-L-serin und Sojaprotein Hydrolysat.

Bevorzugte kosmetische oder pharmazeutische Zubereitungen enthalten neben dem erfindungsgemäßen Extrakt weiterhin einen, zwei, drei oder mehrere Proteasom-Aktivatoren. Vorteilhafte Proteasom-Aktivatoren sind z.B. Phaeodactylum tricornutum, Palmitoylisoleucin, Olea Europaea Blatt Extrakt, Oleuropein, hydrolysierter Candida Saitoana Extrakt (Silab) und Plankton Extrakt aus Scenedesmus von Sahel (Biotech Marine).

Erfindungsgemäß umfassen die kosmetischen und pharmazeutischen Zubereitungen gegebenenfalls ein, zwei, drei, vier oder mehrere weitere antiinflammatorische wirksame Verbindungen. Es können hierbei alle für kosmetische und/oder dermatologische Anwendungen geeigneten oder gebräuchlichen hautirritations- und/oder hautrötungsverringernden Wirkstoffe verwendet werden. Besonders bevorzugt sind jedoch Kombinationen mit einer, zwei, drei, vier oder mehreren weiteren antiinflammatorisch wirksamen Verbindungen ausgewählt aus der Gruppe der folgenden Verbindungen: steroidale entzündungshemmende Substanzen vom Kortikosteroiden-Typ, bevorzugt Hydrocortison, Hydrocortison-Derivate wie Hydrocortison-17-butyrat, Dexamethason, Dexamethasonphosphat, Methylprednisolon oder Cortison sowie weitere steroidale Entzündungshemmer; nichtsteroidale Entzündungshemmer, bevorzugt Oxicame wie Piroxicam oder Tenoxicam; Salicylate wie Aspirin, Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Benoxaprofen oder Pyrazole wie Phenylbutazon, Oxyphenylbutazon, Febrazon oder Azapropazon; natürliche entzündungshermmende bzw. rötungs- und/oder juckreizlindernde Stoffe, bevorzugt Pflanzenextrakte, spezielle hochwirksame Pflanzenextrakt-Fraktionen sowie aus Pflanzenextrakten isolierte hochreine Wirksubstanzen, besonders bevorzugt sind Extrakte, Fraktionen und Wirksubstanzen aus Kamille, Ingwer, Aloe vera, Commiphora-Arten, Süßholz (Glycyrrhiza-)Arten wie Glycyrrhiza glabra und Glycyrrhiza

inflata, Rubia-Arten, Weide, Weidenröschen, Hafer, Calendula, Arnika, Johanniskraut, Geissblatt, Rosmarin, Melisse, Passiflora incarnata, Hamamelis, Pueraria, Dianthus oder Echinacea sowie isolierte Reinsubstanzen hieraus, bevorzugt Bisabolol, Apigenin, Apigenin-7-glucosid, Rosmarinsäure, Boswelliasäure, Phytosterole, Glycyrrhizinsäure, Glabridin, Licochalkon A und Anthranilsäureamide wie insbesondere Avenanthramide oder Dianthramide; natürlich vorkommende Anthranilsäure-Derivate, bevorzugt aus Hafer oder Nelken (Dianthus-Arten); vollsynthetisch oder partialsynthetisch hergestellte Anthranilsäureamide, vorzugsweise Dihydroavenanthramid D (Symrise AG); natürlich vorkommende Inhaltsstoffe von Süßholz und/oder vollsynthetische oder partialsynthetisch hergestellte Verbindungen, bevorzugt Tetrahydrolicochalcon A sowie die in WO 2005/123101 genannten Verbindungen.

Die Menge der Antiirritantien (eine oder mehrere Verbindungen) in den erfindungsgemäßen Zubereitungen beträgt vorzugsweise 0,0001 bis 20 Gew.-%, besonders bevorzugt 0,0001 - 10 Gew.-%, insbesondere 0,001 - 5 Gew.-%, bezogen auf das Gesamtgewicht der Zubereitung.

Der eine bzw. die mehreren weiteren Stoffe mit physiologischer Kühlwirkung, die als Bestandteil in einer erfindungsgemäßen kosmetische, dermatologische und/oder pharmazeutische Zubereitung eingesetzt werden können, werden dabei vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (l-Menthoxy)-1,2-propandiol, 1, (l-Menthoxy)-2-methyl-1,2-propandiol, l-Menthylmethylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (dabei vorzugsweise Menthancarbonsäure-N-ethylamid [WS3] oder $N^{\alpha}$-(Menthancarbonyl)glycinethylester [WS5], wie in US 4,150,052 beschrieben, Menthancarbonsäure-N-(4-cyanophenyl)amid oder Menthancarbonsäure-N-(4-cyanomethylphenyl)amid wie in WO 2005/049553 beschrieben, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (l-(-)-Isopulegol, l-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben).

Der oder die mehreren weiteren Stoffe mit physiologischer Kühlwirkung, die als Bestandteil einer erfindungsgemäßen kosmetische, dermatologische und/oder pharmazeutische Zubereitung eingesetzt werden können, sind insbesondere vorzugsweise Stoffe, welche zumindest im Wesentlichen eine physiologische Kühlwirkung verursachen. Solche bevorzugten Stoffe sind: Menthylether (z.B. (l-Menthoxy)-1,2-propandiol, (l-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (z.B. Menthancarbonsäure-N-ethylamid [WS3], $N^{\alpha}$-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

Komponenten, die ein Wärme-, Schärfe-, Kribbel- oder Prickelgefühl auf der Haut oder auf den Schleimhäuten hervorrufen, insbesondere Aromastoffe mit einem wärmeerzeugenden Effekt und/oder scharf schmeckende Verbindungen (Scharfstoffe), die neben einem erfindungsgemäßen bzw. erfindungsgemäß einzusetzenden *Gleditsia triacanthos* Holzextrakt Bestandteil einer erfindungsgemäßen Zubereitung sein können, sind in WO 2005/123101 genannt.

In manchen Zubereitungen kann auch eine Kombination mit (Metall)-Chelatoren vorteilhaft sein. Bevorzugt einzusetzende (Metall)-Chelatoren sind die in WO 2005/123101 genannten Verbindungen.

Die erfindungsgemäßen bzw. erfindungsgemäß einzusetzenden *Gleditsia triacanthos* Holzextrakte können in kosmetischen und dermatologischen Zubereitungen auch vorteilhaft in Kombination mit Insektrepellents wie z.B. DEET, IR 3225, Dragorepel (Symrise GmbH & Co. KG) eingesetzt werden.

Die erfindungsgemäßen bzw. erfindungsgemäß einzusetzenden *Gleditsia triacanthos* Holzextrakte können ferner in kosmetischen und dermatologischen Zubereitungen vorteilhaft in Kombination mit Haarpflegemitteln und Antischuppenwirkstoffen (z.B. Climbazol, Ketoconazol, Piroctonoleamin, Zink-Pyrithion) eingesetzt werden.

Auch können die erfindungsgemäßen bzw. erfindungsgemäß einzusetzenden *Gleditsia triacanthos* Holzextrakte in zahlreichen Fällen vorteilhaft in Kombination mit einem oder mehreren Konservierungsmitteln in erfindungsgemäßen Zubereitungen eingesetzt werden. Vorzugsweise gewählt werden hierbei die in WO 2005/123101 genannten

Konservierungsmittel.

Erfindungsgemäße Zubereitungen können neben einem erfindungsgemäßen bzw. erfindungsgemäß einzusetzenden *Gleditsia triacanthos* Holzextrakt auch für kosmetische Zwecke verwendbare weitere Pflanzenextrakte enthalten. Vorzugsweise werden die Pflanzenextrakte gewählt aus der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt, beginnenden Tabelle aufgeführten Stoffe. Vorteilhaft sind ferner insbesondere die in WO 2005/123101 genannten Extrakte.

Kosmetische Zubereitungen, enthaltend einen erfindungsgemäßen bzw. erfindungsgemäß einzusetzenden *Gleditsia triacanthos* Holzextrakt können, insbesondere wenn kristalline oder mikrokristalline Festkörper wie beispielsweise anorganische Mikropigmente in die Zubereitungen eingearbeitet werden sollen, erfindungsgemäß auch in WO 2005/123101 genannte anionische, kationische, nichtionische und/oder amphotere Tenside enthalten.

Die oberflächenaktive Substanz kann in einer Konzentration zwischen 1 und 98 Gew.-% in den erfindungsgemäßen Zubereitungen vorliegen, bezogen auf das Gesamtgewicht der Zubereitungen.

Die Ölphase einer erfindungsgemäßen Zubereitung enthaltend einen erfindungsgemäßen bzw. erfindungsgemäß einzusetzenden *Gleditsia triacanthos* Holzextrakt kann vorteilhaft gewählt werden aus den in WO 2005/123101 genannten Substanzgruppen.

Ferner vorteilhaft sind erfindungsgemäße Zubereitungen, die oral verabreicht werden, z.B. in Form von Tabletten (z.B. Filmtabletten), Dragees, Kapseln (z.B. Gelatinekapseln), Granulaten, Säften, Lösungen, Emulsionen, Mikroemulsionen, Sprays oder in sonstiger Form oral konsumierbarer Produkte oder in Form von Lebensmitteln.

Die Erfindung betrifft ferner einen erfindungsgemäßen Holzextrakt, vorzugsweise in einer der als bevorzugt gekennzeichneten Ausgestaltungen, sowie eine erfindungsgemäße Mischung, vorzugsweise in einer der als bevorzugt gekennzeichneten Ausgestaltungen, zur Anwendung in einem Verfahren oder zur Verwendung zur Vorbeugung, Behandlung und/oder Verringerung von Cellulite.

Die Erfindung betrifft auch einen erfindungsgemäßen bzw. erfindungsgemäß einzusetzenden Holzextrakt, vorzugsweise in einer der als bevorzugt gekennzeichneten Ausgestaltungen, eine erfindungsgemäße Mischung, vorzugsweise in einer der als bevorzugt gekennzeichneten Ausgestaltungen, sowie eine erfindungsgemäße Zubereitung, vorzugsweise in einer der als bevorzugt gekennzeichneten Ausgestaltungen, als Arzneimittel.

Wie oben bereits ausgeführt, zeigen die erfindungsgemäßen bzw. erfindungsgemäß einzusetzenden *Gleditsia triacanthos* Holzextrakte, vorzugsweise in den als bevorzugt gekennzeichneten Ausgestaltungen, zusätzlich eine SIRT-stimulierende, d.h. SIRT-aktivierende, Wirksamkeit, insbesondere bezüglich SIRT1. Aufgrund ihrer SIRT-stimulierenden Eigenschaften wirken die erfindungsgemäßen bzw. erfindungsgemäß einzusetzenden *Gleditsia triacanthos* Holzextrakte auch als Wirkstoffe zur Vorbeugung, Behandlung und/oder Verringerung der Alterung, insbesondere der Hautalterung, zur Verbesserung der Stressantwort und Vorbeugung, Verringerung und/oder Reparatur von stressbedingten Schädigungen und/oder zur Erlangung eines gleichmäßigen Hauttons.

Wie oben bereits ausgeführt, zeigen die erfindungsgemäßen bzw. erfindungsgemäß einzusetzenden *Gleditsia triacanthos* Holzextrakte, vorzugsweise in den als bevorzugt gekennzeichneten Ausgestaltungen, zusätzlich eine anti-irritierende Wirksamkeit. Aufgrund ihrer entzündungshemmenden Eigenschaften, insbesondere in Bezug auf die Hemmung der COX-2 Aktivität, wirken die erfindungsgemäßen bzw. erfindungsgemäß einzusetzenden *Gleditsia* Holzextrakte auch als Wirkstoffe zur Vorbeugung, Behandlung und/oder Verringerung von Hautirriationen und Hautentzündungen und/oder zur Erlangung eines gleichmäßigen Hauttons.

Der Begriff "Haut" beinhaltet im Rahmen dieser Erfindung im Zusammenhang mit der SIRT-stimulierenden und/oder der anti-irritierenden Wirksamkeit eiens erfindungsgemäßen bzw. erfindungsgemäß einzusetzenden *Gleditsia* Holzextraktes auch "Schleimhaut" (Mucosa), insbesondere die Schleimhaut von Mund, Zahnfleisch, Hals, Rachen, Nase, Respirations- und gastrointestinal Trakt.

Neben kosmetischen und dermatologischen Nutzen, bieten sich SIRT Aktivatoren auch als pharmazeutische (therapeutische) Wirkstoffe an. Krankheiten wie chronische und degenerative Erkrankungen, kardiovaskuläre Erkrankungen, maligne Neoplasien, Lebererkrankungen, Diabetes mellitus, Arthritis, inflammatorische Erkrankungen und Alzheimer nehmen mit dem Alter zu. Neuere Studien bringen Sirtuine und insbesondere SIRT1 mit vielen dieser Alterserkrankungen in Verbindung.

So konnte für altersbedingte Herzerkrankungen gezeigt werden, dass die Überexpression von SIRT1 beispielsweise zu einer Verringerung der Herzhypertrophyie, Apoptosis, Fibrosis, Herzdysfunktion und der Expression von Seneszenzmarkern führt. Auch unterstützt SIRT indirekt die Herzfunktion durch die Deacetylierung des eNOS Enzyms.

SIRT1 kann weiterhin Fettvorräte des Körpers durch die Unterdrückung des PPARgamma Rezeptors mobilisieren. Dieser Effekt, zusammen mit niedriger Insulinproduktion, erhöhter Insulinsensivität und dem Insulin Growth Factor 1 (IGF-1), kann alterabhängige, mit Fettleibigkeit in Zusammenhang stehende Krankheiten verzögern, wie z.B. Diabetes.

Auch spielt SIRT1 offenbar eine Rolle in der Alzheimer-Pathologie, indem es die Produktion von β-Amyloid, das die alzheimertypischen Plaques bildet, kontrolliert.

Im Folgenden wird die Erfindung anhand von Beispielen weiter erläutert. Die Beispiele dienen zur Verdeutlichung der Erfindung, ohne den Schutzbereich der Patentansprüche einzuschränken. Sofern nicht anders angegeben, beziehen sich alle Angaben auf das Gewicht.

**Beispiel 1: Herstellung von Christusdorn Kernholzextrakten im Labormaßstab**

Zur Herstellung der erfindungsgemäßen Holzextrakte wurde Holzraspat aus Christusdorn (*Gleditsia triacanthos*) Kernholz (Charge 1 bzw. 2 aus zwei verschiedenen Holzstämmen) mit dem in nachfolgender Tabelle 1.1 jeweils angegebenen Extraktionsmittel in dem jeweils angegebenen Mengenverhältnis versetzt und 2 h unter Rückfluss bei Umgebungsdruck (Labor) extrahiert. Anschließend wurde das Gemisch abgekühlt, filtriert und das bzw. die Extraktionsmittel sowie Wasser im Vakuum vollständig entfernt, so dass ein Trockenextrakt enthalten wurde.

Tabelle 1.1: Erfindungsgemäße Christusdorn (*Gleditsia triacanthos*) Kernholzextrakte

| Testprobe | Extraktionsmittel | Holz-Charge | Gewichtsverhältnis Holzraspat : Extraktionsmittel | Ausbeute an Trockenextrakt [Gew.-%] |
|---|---|---|---|---|
| BIO2162 | Wasser | 2 | 1 : 12 | 3,3 |
| BIO2162/1 | Wasser | 1 | 1 : 17 | 3,4 |
| BIO781/1 | Ethanol/Wasser 1: 1 (m/m) | 2 | 1 : 8 | 5,9 |
| BIO781/2 | Ethanol/Wasser 1: 1 (m/m) | 1 | 1 : 21 | 6,2 |
| BIO2163 | Ethanol | 2 | 1 : 12 | 2,8 |
| BIO2163/1 | Ethanol | 1 | 1 : 17 | 2,3 |
| BIO792 | Essigester | 1 | 1 : 21 | 1,0 |
| BIO796 | Aceton | 1 | 1 : 16 | 2,3 |

Die Charakterisierung der Extrakte erfolgt durch HPLC-Fingerprint-Analyse und quantitative Bestimmung des Gehaltes an Fisetin (3,3',4',7-Tetrahydroxyflavon) und Fustin (3,3',4',7-Tetrahydroxyflavanon).

HPLC-Säule: YMC ODS-AQ, 5 μm, 150x3 mm mit Vorsäule, Temperatur: 40°C, Fluss: 0,6 ml/min, Acetonitril/Wasser mit 0,1% Ameisensäure-Gradient, Injektionsvolumen: 5 μl, Detektionswellenlängen: 232 und 280 nm

Tabelle 1.2: Gehalt (bezogen auf den Trockenextrakt) an Fisetin und Fustin in verschiedenen Christusdorn Kernholzextrakten

| Testprobe | Extraktionsmittel | Holz-Charge | Fisetingehalt [Gew.-%] | Fustingehalt [Gew.-%] |
|---|---|---|---|---|
| BIO216 | Wasser | 2 | Nicht detektierbar | 5,3 |
| BIO2162/1 | Wasser | 1 | Nicht detektierbar | 0,9 |
| BIO781/1 | Ethanol/Wasser 1:1 (m/m) | 2 | 0,9 | 11,1 |
| BIO781/2 | Ethanol/Wasser 1:1 (m/m) | 1 | 1,0 | 1,5 |
| BIO2163 | Ethanol | 2 | 1,8 | 17,4 |
| BIO2163/1 | Ethanol | 1 | 2,1 | 3,5 |
| BIO792 | Essigester | 1 | 2,4 | 1,8 |
| BIO796 | Aceton | 1 | 4,7 | 1,4 |

**Beispiel 1A: Herstellung eines erfindungsgemäßen *Gleditsia* Holzextraktes im technischen Maßstab**

Holzraspat von *Gleditsia triacanthos* wurde in einem Siebkorbextraktor mit dem Extraktionsmittel = EtOH/Wasser 1:1 (m/m) (Massenverhältnis Holzraspat : Extraktionsmittel = 1: 7) über einen Zeitraum von 2 h unter Rückfluss extrahiert. Der resultierende erste Extrakt wurde abgetrennt und das Holz ein zweites Mal unter gleichen Bedingungen extrahiert. Die beiden so erhaltenen Extrakte wurden vereint und danach destillativ eingeengt, so dass das erhaltene wässrige Produkt weitgehend frei von Ethanol war (der entsprechende Trockenextraktgehalt des resultierenden Produktes lag bei etwa. 7,5 Gew.-%).

**Beispiel 1B: Herstellung einer erfindungsgemäßen Mischung**

| | |
|---|---|
| 1,2-Propylenglycol | 60 Gew.-% |
| Wasser | ad 100 Gew.-% |
| *Gleditsia triacanthos* Kernholzextrakt aus Beispiel 1A | 3 Gew.-% |
| Natriumbenzoat | 0,5 Gew.-% |
| Kaliumsorbat | 0,2 Gew.-% |

Milchsäure zum Einstellen des pH-Wertes auf 4,5

Das Produkt aus Beispiel 1A wurde mit Propylenglycol, Wasser, Natriumbenzoat und Kaliumsorbat in der jeweils angegebenen Menge vermischt und der pH-Wert dieser Mischung auf einen Wert von 4,5 (gemessen bei 25°C) eingestellt, so dass eine erfindungsgemäße Mischung mit obiger Zusammensetzung erhalten wurde. Nach 5 Tagen Standzeit wurde diese Mischung filtriert.

**Beispiel 2: Adipogenese-Assay**

3T3-L1-Zellen (Maus embryonale Fibroblasten-ähnliche Adipozyten-Zelllinie) werden in eine 48-well-Platte mit Collagen I-Beschichtung in einer Konzentration von 3 x $10^4$ Zellen / Well ausgesät. Nach 72 h Kultivierung bei 37 °C und 5% $CO_2$ in DMEM (Dulbecco's Modified Eagle Medium), angereichert mit 10% Kälberserum, werden verschiedene Konzentrationen der Testproben in DMEM, angereichert mit 10% fötalem Kälberserum sowie versetzt mit 1 $\mu$g/ml Insulin, 0,25 $\mu$M Dexamethason und 0,5 mM IBMX (3-Isobutyl-1-Methylxanthin), zugegeben und weitere 48 h inkubiert. Es erfolgt ein Medienwechsel, indem DMEM, angereichert mit 10% fötalem Kälberserum sowie versetzt mit 1 $\mu$g/ml Insulin, aufgetragen wird. Nach erneuter Kultivierung für 48 h erfolgt ein weiterer Medienwechsel, bei dem DMEM, angereichert mit 10% fötalem Kälberserum, aufgetragen wird.

Nach weiterer Inkubation für 72 h erfolgt die Quantifizierung der intrazellulär eingelagerten Lipide als Maß für die Differenzierung der Zellen durch Messung der Fluoreszenz nach Anfärbung der Lipide mit dem Fluoreszenz-Farbstoff Nilrot (Nile Red).

Die Inhibition der Adipogenese in Anwesenheit der Testproben wird gemäß der folgenden Gleichung berechnet:

$$\text{Inhibition der Adipogenese}\,[\%] = 100 - \left( \frac{\text{RFU Testsubstanz} - \text{RFU Kontrolle ohne Zellen}}{\text{RFU Kontrolle} - \text{RFU Kontrolle ohne Zellen}} \times 100 \right)$$

mit

RFU Testsubstanz = relative Fluoreszenz-Einheiten der Wells mit Testsubstanz und mit Zellen

RFU Kontrolle = relative Fluoreszenz-Einheiten der Wells ohne Testsubstanz, aber mit Zellen

RFU Kontrolle ohne Zellen = relative Fluoreszenz-Einheiten der Wells ohne Testsubstanz und ohne Zellen.

Aus der Adipogenese-Inhibition [%] in einer Reihe von Verdünnungen von getesteten Proben wird der $IC_{50}$ berechnet. Dies ist die Konzentration, bei der die Adipogenese zu 50 % gehemmt wird.

Tabelle 2.1: Adipogenese-Inhibition verschiedener Christusdorn Kernholzextrakte (Mittelwerte aus mindestens 2 unabhängigen Versuchen)

| Testprobe | $IC_{50}$ [%] |
|---|---|
| BIO781/2 | 0,011 |
| BIO792 | 0,006 |

Die Extrakte zeigen eine deutliche Hemmung der Adipogenese.

Um den Einfluss der beiden identifizierten Inhaltsstoffe Fustin und Fisetin zu untersuchen wurden diese parallel zu dem Extrakt in der ihrem Gehalt im Extrakt entsprechenden Konzentration getestet.

Tabelle 2.2: Adipogenese-Inhibition von Christusdorn Kernholzextrakt BIO781/2 und den beiden darin enthaltenen Stoffen Fustin und Fisetin

| Testprobe | Gehalt in BIO781/2 [Gew.-%] | Testkonzentration [%] | Adipogenese-Hemmung [%] |
|---|---|---|---|
| BIO781/2 | | 0,03 | 40 |
| Fustin | 1,5 | 0,0005 | Keine Hemmung |
| Fisetin | 1,0 | 0,0003 | Keine Hemmung |

Die Ergebnisse zeigen, dass Fustin und Fisetin in der getesteten Konzentration keine Aktivität zeigen und daher für die Adipogenese hemmende Wirkung des Extrakts BIO781/2 nicht verantwortlich sind.

**Beispiel 3: Lipogenese-Assay**

Unter Lipogenese wird die Einlagerung von Triglyceriden in Adipozyten verstanden. Die Hemmung dieser Einlagerung kann durch die Hemmung der Aktivität extrazellulärer Lipoprotein-Lipase (LPL) erfolgen, indem die Hydrolyse extrazellulärer Triglyceride und dadurch die Aufnahme freier Fettsäuren durch Adipozyten reduziert wird. Als Vorversuch wird die Hemmung von pankreatischer Lipase (PL) untersucht.

PL (Sigma-Aldrich) wird in Anwesenheit von Prüfsubstanzen in unterschiedlichen Einsatzkonzentrationen mit Methylumbelliferyl-Oleat (MUF-Oleat) als Substrat versetzt. Durch Hydrolyse des MUF-Oleats durch PL entsteht fluoreszierendes Methylumbelliferon (MUF), das quantifiziert wird. Die Hemmung der Hydrolyse des MUF-Oleats ist ein Maß für die Hemmung der Aktivität der PL.

$$\text{Inhibition der PL } [\%] = 100 - \left( \frac{\text{MUF Testsubstanz} - \text{MUF Kontrolle ohne PL}}{\text{MUF Kontrolle} - \text{MUF Kontrolle ohne PL}} \times 100 \right)$$

mit

MUF Testsubstanz = MUF-Konzentration der Wells mit Testsubstanz und mit PL
MUF Kontrolle = MUF-Konzentration der Wells ohne Testsubstanz, aber mit PL
MUF Kontrolle ohne PL = MUF-Konzentration der Wells ohne Testsubstanz und ohne PL.

Aus der Inhibition der PL [%] in einer Reihe von Verdünnungen von getesteten Proben wird der $IC_{50}$ berechnet. Dies ist die Konzentration, bei der die Aktivität der PL zu 50 % gehemmt wird.

Tabelle 3: Inhibition der PL durch verschiedene Christusdorn Kernholzextrakte

| Testprobe | $IC_{50}$ [%] |
|---|---|
| BIO781/2 | 0,054 |
| BIO792 | 0,012 |
| BIO796 | 0,013 |

Die Extrakte zeigen eine deutliche Hemmung der Lipaseaktivität.

**Beispiel 4: SIRT1 Assay**

NHDF Zellen (normale humane dermale Fibroblasten) werden in eine 96-well Mikrotiterplatte in ausgesät. Die Zellen werden 24 h bei 37 °C und 5% $CO_2$ in DMEM (Dulbecco's Modified Eagle Medium) kultiviert. Anschließend werden die zu testenden Proben zugegeben und die Zellen werden für weitere 48 h kultiviert. Nach Waschen der Zellen mit PBS (phosphatgepufferte Salzlösung) werden die Zellen mit Paraformaldehyd fixiert und permeabilisiert. Anschließend werden sie noch einmal gewaschen, mit BSA (Bovinem Serumalbumin) blockiert und mit einem Sekundärantikörper inkubiert. Nach extensivem Waschen wird die Fluoreszenz mit einem Mikroplatten-Reader gemessen und Fluoreszenzbilder über eine an ein Fluoreszenzmikroskop gekoppelte Kamera aufgenommen.

Die Stimulierung der SIRT1 Proteinexpression in Anwesenheit der Testproben wird gemäß der folgenden Gleichung berechnet:

$$\text{Stimulierung der SIRT1 Pr}\mathit{oteine}\text{xpression [\%]} = \left( \frac{\text{RFU Testprobe - RFU Hintergrund}}{\text{RFU Kontrolle - RFU Hintergrund}} \text{ x } 100 \right) - 100$$

RFU Testprobe = Relative Fluoreszenzeinheiten der Wells mit Testprobe, komplett angefärbt

RFU Kontrolle = Relative Fluoreszenzeinheiten der Wells ohne Testprobe, komplett angefärbt

RFU Hintergrund = Relative Fluoreszenzeinheiten der Wells ohne Testprobe, nur mit Sekundärantikörper angefärbt.

Tabelle 4: Stimulierung der SIRT1 Proteinexpression durch Christusdornholz Ethanol/Wasser 1:1 Extrakte versus Kontrolle

| Testprobe | Konzentration [%] | Stimulation [%] |
|---|---|---|
| BIO781/1 | 0,01 | 25 |
| BIO781/2 | 0,01 | 21 |

Die beiden Testproben zeigen eine statistisch signifikante ($p < 0,05$, T-Test) Stimulierung der SIRT1 Proteinexpression. Obwohl sich beide Extrakte deutlich hinsichtlich ihres Gehaltes an Fustin sowie bezüglich ihres HPLC-Fingerprints unterscheiden, ist der stimulierende Effekt auf die SIRT1 Proteinexpression vergleichbar.

**Beispiel 5: Cyclooxgenase-2 (COX-2)-Assay**

Es wurde das COX Inhibitionsscreening Assay der Cayman Chemical Company verwendet. COX-2 wird in Gegenwart der Testproben mit dem fluorometrischen Substrat 10-Acetyl-3,7-dihydroxyphenoxanin (ADHP) und Häm gemischt. Die Reaktion wird durch Zugabe des Substrats Arachidonsäure gestartet.

COX-2 konvertiert die Arachidonsäure zu Prostaglandinendoperoxid G2 (PGG2). Anschließend wird PGG2 zu dem entsprechenden Alkohol PGH2 reduziert. Während dieser Reaktion wird aus ADHP das fluoreszierende Resorufin. Resorufin wird bei einer Extinktionswellenlänge von 535 nm und einer Emissionswellenlänge von 590 nm quantifiziert.

Inhibition der COX-2 [%] = 100 – ((Resorufin Testprobe – Resorufin Kontrolle ohne COX-2 / Resorufin Kontrolle – Resorufin Kontrolle ohne COX-2) X 100)

mit

Resorufin Testprobe = Resorufin Konzentration der Wells mit Testprobe und mit COX-2
Resorufin Kontrolle = Resorufin Konzentration der Wells ohne Testprobe, aber mit COX-2
Resorufin Kontrolle ohne COX-2 = Resorufin Konzentration der Wells ohne Testprobe und ohne COX-2.

Aus der Inhibition der COX-2 [%] in einer Reihe von Verdünnungen von getesteten Proben wird der $IC_{50}$ berechnet. Dies ist die Konzentration, bei der die Aktivität der COX-2 zu 50 % gehemmt wird.

Tabelle 5: Inhibition der COX-2 durch verschiedene Christusdorn Kernholzextrakte

| Testprobe | $IC_{50}$ [%] |
|---|---|
| BIO781/2 | 0,0005 |
| BIO781/1 | 0,0011 |
| BIO2162 | 0,0021 |
| BIO2163 | 0,0010 |

Alle Holzextrakte zeigten eine deutliche Hemmung der COX-2 Aktivität. Aus den Ergebnissen lässt sich ableiten, dass Fisetin und Fustin nicht bzw. lediglich zu einem geringen Teil für die entzündungshemmende Wirkung der Extrakte verantwortlich sind. So zeigt zum einen Extrakt BIO2162 gute Aktivität, obwohl Fisetin nicht nachweisbar ist und zum anderen weist BIO781/1 trotz des um Faktor 7 höheren Fustingehalts geringere COX-2 inhibierende Aktivität auf als BIO781/2.

**FORMULIERUNGSBEISPIELE:**

| Beispiel Nr. | Produktform |
|---|---|
| 1 | Antischuppenshampoo |
| 2 | Anti-Cellulite Körperöl |
| 3 | After-Sun Spray O/W |
| 4 | Hautstraffende Nachtcreme W/O |
| 5 | Antifalten Ampulle |
| 6 | After-Shave Hydrogel |
| 7 | Körperpeeling |
| 8 | Anti-Cellulite Balm |
| 9 | Hautaufhellendes Tagespflegefluid O/W |
| 10 | Barrierreperatur Creme O/W |
| 11 | Sonnenschutz Lotion SPF 24 (UVA/UVB Balance) |

| Inhaltsstoffe | INCI-Name | Gew.-% | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Gleditsia triacanthos Holzextrakt, getrocknet | Gleditsia triacanthos Extract | | | | | 0,01 | | | | | | |
| Gleditsia triacanthos Holzextrakt (Trockenextraktgehalt 3%) | Propylenglycol, Water (Aqua), Gleditsia triacanthos Extract | | | 3 | | | | 2 | | | | 0,5 |
| Gleditsia triacanthos Holzextrakt (Trockenextraktgehalt 10%) | Maltodextrin, Gleditsia triacanthos Extract | 0,1 | | | | | | | 0,5 | 2 | | |
| Gleditsia triacanthos Holzextrakt (Trockenextraktgehalt 1%) | Caprylic/Capric Triglyceride, Gleditsia triacanthos Extract | | 5 | | | | | | | | 1 | |
| A-C Polyethylen 9 A | Polyethylene | | | | | | | 5 | | | | |
| Actipone Black Coffee GW | Water (Aqua), Glycerin, Coffea Arabica (Coffee) Seed Extract, Coffea Robusta Seed Extract | | | | | | | | 0,5 | | | |
| Actipone Laminaria Saccharina | Glycerin, Water (Aqua), Laminaria Saccharina Extract | | | | | | 0,3 | | | | | |
| Actipone Nutgrass (Motha) Root GW | Water (Aqua), Glycerin, Cyperus Rotundus Root Extract | | | | | | | 0,2 | | | | |
| Allantoin | Allantoin | | | | | | 0,1 | | | | | |
| Aloe Vera Gel Conc. 10:1 | Aloe Barbadensis Leaf Juice | | | | | | | | | | | 1 |
| Aluminium Stearate | Aluminium Stearate | | | | 1,2 | | | | | | | |
| Arbutin | β-Arbutin | | | | | | | | | 1,0 | | |
| Arlypon F | Laureth-2 | 2 | | | | | | | | | | |
| Avocado Öl | Persea Gratissima (Avocado) Oil | | 2 | 3 | | | | | | | | |
| Biotive L-Arginine | Arginine | | | | | | | | | | | 0,5 |
| Biotive Esculin Sesquihydrate | Esculin | | | | | | | | 0,1 | | | |
| Biotive Troxerutin | Troxerutin | | | | | | | | | | | 0,5 |
| (-)-alpha-Bisabolol natürlich | Bisabolol | | | 0,2 | | | | | 0,1 | | 0,2 | 0,1 |
| Carbopol Ultrez-10 | Carbomer | | | | | | 0,4 | | | 0,2 | | |

(fortgesetzt)

| Inhaltsstoffe | INCI-Name | Gew.-% | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Carnitin | Carnitine | | | | | | | | 0,8 | | | |
| CeramideBIO | Cetylhydroxyproline Palmitamide | | | | | | | | | | 0,5 | |
| Citronensäure 10%in Wasser | Citric Acid | 0,5 | | | | | | 0,05 | 0,2 | | | |
| Covi-Ox T-70 | Tocopherol | | 0,2 | 0,1 | | | | | | | | |
| Crinipan AD | Climbazole | 0,3 | | | | | | | | | | |
| D-Panthenol | Panthenol | 0,5 | | 1 | | 1 | 0,5 | 0,5 | | | | |
| Dermacryl AQF | Acrylates Copolymer | | | | | | | | | | | 2 |
| Dow Corning 200(100cs) Silicone Fluid | Dimethicone | | | | | | | | 3 | 0,5 | 0,5 | |
| Dow Corning 246 Fluid | Cyclohexasiloxane, Cyclopentasiloxane | | | 2 | | | | | | | | 3 |
| Dracorin 100 S.E.P. | Glyceryl Stearate, PEG-100 Stearate | | | | | | | 7 | | | | |
| Dracorin CE | Glyceryl Stearate Citrate | | | | | | | | | | 1,5 | |
| Dracorin GMS | Glyceryl Stearate | | | | | | | | | | 2 | |
| Dracorin GOC | Glyceryl Oleate Citrate, Caprylic/Capric Triglyceride | | | 2 | | | | | | | | |
| DragoCalm | Water (Aqua), Glycerin, Avena Sativa (Oat) Kernel Extract | | | | | 1 | | | | | | |
| Dragocid Liquid | Phenoxyethanol, Methylparaben, Ethylparaben, Butylparaben, Propylparaben, Isobutylparaben | 0,7 | | | 0,8 | | | | 0,4 | 0,8 | 0,8 | |
| Dragoderm | Glycerin, Triticum Vulgare (Wheat) Gluten, Water (Aqua) | 0,5 | | | | | | | | | | |
| Dragosan W/O P | Sorbitan Isostearate, Hydrogenated Castor Oil, Ceresin, Beeswax (Cera Alba) | | | | 8 | | | | | | | |
| Dragosantol 100 | Bisabolol | | | | 0,2 | | 0,1 | | | | | |
| Dragosine | Carnosine | | | | | 0,2 | | | | | | |

(fortgesetzt)

| Inhaltsstoffe | INCI-Name | Gew.-% | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Dragoxat 89 | Ethylhexyl Isononanoate | | 10 | | 7 | | | | | | 2 | 2 |
| Edeta B Powder | Tetrasodium EDTA | | | | | | | 0,1 | | | | |
| Edeta BD | Disodium EDTA | | | | | | | | | 0,1 | | 0,1 |
| Emulgin B2 | Ceteareth-20 | | | | | | | 2 | | | | |
| Emulsiphos | Potassium Cetyl Phosphate, Hydrogenated Palm Glycerides | | | | | | | | | 1,5 | 2 | 2 |
| Ethanol | Alcohol Denat. | | | 5 | | | 8 | | | | | |
| Etherisches Öl | Essential Oil | | 0,2 | | | | | | | | | |
| Extrapone Aloe vera | Water (Aqua), Aloe Barbadensis, Propylene Glycol, Alcohol | | | | | | 2 | | | | | |
| Extrapone Butcher's broom GW P | Glycerin, Water (Aqua), Pentylene Glycol, Ruscus Aculeatus Root Extract | | | | | | | 1 | | | | |
| | | 1 | 2 | 3 | 4 | 5 | 6 | 7 | 8 | 9 | 10 | 11 |
| Extrapone Ginkgo Biloba | Propylene Glycol, Water (Aqua), Ginkgo Biloba Leaf Extract, Glucose, Lactic Acid | | | | | | | 0,3 | | | | |
| Extrapone Green Tea (Organic) GW | Water (Aqua), Glycerin, Camellia Sinensis Leaf Extract | | | | | | | 2 | | | | |
| Extrapone Guarana | Water (Aqua), Propylene Glycol, Paullinia Cupana Seed Extract, Alcohol | 0,5 | | | | | | | | | | |
| Extrapone Horse Chestnut | Propylene Glycol, Water (Aqua), Aesculus Hippocastanum (Horse Chestnut) Seed Extract, Glucose, Lactic Acid | | | | | | | 1 | | | | |
| Extrapone Ivy | Propylene Glycol, Water (Aqua), Hedera Helix (Ivy) Leaf/Stem Extract, Glucose, Lactic Acid | | | | | | | 0,2 | | | | |
| Extrapone Orange Flower | Water (Aqua), Propylene Glycol, Citrus Aurantium Amara (Bitter Orange) Flower Extract | | | | | | | 1,0 | | | | |

(fortgesetzt)

| Inhaltsstoffe | INCI-Name | Gew.-% | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Extrapone Orange Peel | Water (Aqua), Propylene Glycol, Alcohol, Citrus Aurantium Dulcis (Orange) Peel Extract | | | | | | | 1 | | | | |
| Extrapone Seaweed | Water (Aqua), Butylene Glycol, Fucus Vesiculosus Extract | | | | | | | | 2,5 | | | |
| Frescolat MAG | Menthone Glycerin Acetal | | | | | 0,1 | | | | | | |
| Frescolat ML | Menthyl Lactate | 0,2 | | 0,5 | | | 0,3 | | | | | |
| Genapol LRO Liquid | Sodium Laureth Sulfate | 37 | | | | | | | | | | |
| Givobio GZN | Zinc Gluconate | | | | | | | | | | 0,5 | |
| Glycerin | Glycerin | | | 4 | 3 | | | | | 3,5 | 3 | 3 |
| Hydrolite 5 | Pentylene Glycol | | | 5 | | | 5 | 5 | | | | 2 |
| Hydroviton-24 | Water (Aqua), Pentylene Glycol, Glycerin, Lactic Acid, Sodium Lactate, Serine, Urea, Sorbitol, Sodium Chloride, Allantoin | | | | 1 | | | | | | 1 | |
| Isoadipate | Diisopropyl Adipate | | | | | | | | | 2 | | |
| Isodragol | Trüsononanoin | | 13 | | | | | | | | 3 | 2 |
| Isopropylpalmitat | Isopropyl-Palmitate | | | | | | | 3 | | | | |
| Jojoba Öil | Simmondsia Chinensis (Jojoba) Seed Oil | | | | 2 | | | | | | | |
| Jojoba Oil Ethoxilate (Oxypon 328) | Peg-26 Jojoba Acid, Peg-26 Jojoba Alcohol | | | | | | | | 1 | | | |
| Kaliumsorbat | Potassium Sorbate | | | 0,1 | | | | | | | | |
| Karion F | Sorbitol | | | | | | | 1 | | | | |
| Keltrol CG-RD | Xanthan Gum | | | | | 0,05 | | | | 0,2 | | 0,4 |
| Kojisäure | Kojic Acid | | | | | | | | | 0,5 | | |
| Koffein | Caffeine | | | | | | | | 0,5 | | | |
| Lanette 16 | Cetyl Alcohol | | | | | | | 3 | | 1,5 | | 1 |
| Lanette O | Cetearyl Alcohol | | | | | | | | | | 2 | 0,5 |

(fortgesetzt)

| Inhaltsstoffe | INCI-Name | Gew.-% | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Lara Care A-200 | Galactoarabinan | | | | | | | | | | | 0,3 |
| Magnesium Sulfate | Magnesium Sulfate | | | | 0,7 | | | | | | | |
| Makadamia-Nuss-Öl | Macadamia Ternifoia Seed Oil | | 0,5 | | | | | | | | | |
| Meersalz (Totes Meer) | Sea Salt (Maris Sal) | | | | | | | 1,5 | | | | |
| Mineralöl | Mineral oil | | | | 8 | | | | | | | |
| Natriumascorbylphosphat | Sodium Ascorbyl Phosphate | | | | | | | | | 1 | | |
| Natriumchlorid | Sodium Chloride | 0,1 | | | | | | | | | | |
| Natriumhydroxid Lsg. 10%ig in Wasser | Sodium Hydroxide | | | | | | 0,75 | | | 0,2 | 0,3 | |
| Neo Actipone White Tea | Camellia Sinensis Leaf Extract | | | | | | | 0,1 | | | | |
| Neo Heliopan 303 | Octocrylene | | | | | | | | | | | 10 |
| Neo Heliopan 357 | Butylmethoxydibenzoylme thane | | | | | | | | | 2 | | 3 |
| Neo Heliopan AP, 15% Lösung, neutralisiert mit L-Arginin | Aqua, Disodium Phenyl Dibenzimidazole Tetrasulfonate, Arginin | | | | | | | | | | | 6,7 |
| Neo Heliopan AV | Ethylhexyl Methoxycinnamate | | | | | | | | | 7,5 | | |
| Neo Heliopan BB | Benzophenone-3 | | | | | | | | | 3 | | |
| Neo Heliopan E 1000 | Isoamyl p.Methoxycinnamate | | | | | | | | | | | 1 |
| Neo Heliopan HMS | Homosalate | | | | | | | | | 10 | | 5 |
| Neo Heliopan Hydro, 20% Lösung, neutralisiert mit Biotive Arginine | Aqua, Phenylbenzimidazole, Sulphonic Acid, Arginin | | | | | | | | | | | 10 |
| Neo Heliopan OS | Ethylhexyl Salicylate | | | | | | | | | 5 | | |
| Neo-PCL Water Soluble N | Trideceth-9, PEG-5 Ethylhexanoate, Water (Aqua) | 1,5 | | | | | 1 | | | | | |
| Neutralöl | Caprylic/Capric Triglyceride | | | 5 | | | | 10 | | | 10 | |

(fortgesetzt)

| Inhaltsstoffe | INCI-Name | Gew.-% | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Oxynex K Liquid | PEG-8, Tocopherol, Ascorbyl Palmitate, Ascorbic Acid, Citric Acid | | | | | | | 0,1 | | | | |
| Ozokerite Wax 2389 | Ozokerite | | | | 2 | | | | | | | |
| Paraffinöl | Paraffinum Liquidum | | ad 100 | | | | | | | | | |
| Parfümöl | Parfum (Fragrance) | 0,5 | 0,5 | 0,25 | 0,3 | 0,1 | 0,1 | 0,4 | 0,4 | 0,3 | 0,1 | 0,2 |
| PCL-Liquid 100 | Cetearyl Ethylhexanoate | | 21 | 4 | 5 | | | | | | | |
| PCL-Solid | Stearyl Heptanoate, Stearyl Caprylate | | | 0,5 | | | | | | | | |
| Pemulen TR-2 | Acrylates/C10-30 Alkyl Acrylate Crosspolymer | | | 0,25 | | | | | | | | |
| Phytoconcentrole Shea Butter | Glycine Soja (Soybean) Oil, Butyrospermum Parkii (Shea Butter) | | 0,5 | | | | | | | | | |
| Polymer JR 400 | Polyquaternium-10 | 0,4 | | | | | | | | | | |
| Propylenglycol-1,2 | Propylene Glycol | | | | | | 5 | | 2 | | | |
| RonaCare Nicotinamide | Niacinamide | | | | | | | | 0,1 | | | |
| Sepigel 305 | Polyacrylamide, C 13-14 Isoparaffin, Laureth-7 | | | | | | | | 2 | | | |
| Silcare Silicone 41 M65 | Stearyl Dimethicone | | | | | | | | | | | 1 |
| Solubilizer | PEG-40 Hydrogenated Castor Oil, Trideceth-9, Propylene Glycol, Water (Aqua) | | | | | 1,5 | 1,3 | | | | | |
| SymCalmin | Butylene Glycol, Pentylene Glycol, Hydroxyphenyl Propamidobenzoic Acid | 0,1 | | | | | | | | | | |
| SymClariol | Decylene Glycol | | | | | | | | | 0,3 | | |
| Symdiol 68 | 1.2-Hexanediol, Caprylyl Glycol | | 1 | | | 1 | | | | | | |
| SymGlucan | Water (Aqua), Glycerin, Beta-Glucan | | | 1 | | 5 | | | | | | 2 |
| SymMatrix | Maltodextrin, Rubus Fruticosus (Blackberry) Leaf Extract | | | | | 0,1 | | | | | | |

(fortgesetzt)

| Inhaltsstoffe | INCI-Name | Gew.-% | | | | | | | | | | |
|---|---|---|---|---|---|---|---|---|---|---|---|---|
| | | 1 | 2 | 3 | 4 | 5 | 6 | 7 | 8 | 9 | 10 | 11 |
| SymMollient W/S | Trideceth-9, PEG-5 Isononanoate, Water (Aqua) | | | | | 2 | | | | | | |
| SymPeptide 222 | Glycerin, Water (Aqua), Myristoyl Pentapeptide-8 | | | | | | | | 5 | | | |
| SymRelief | Bisabolol, Zingiber Officinale (Ginger) Root Extract | 0,2 | 0,1 | | | | | | | | | 0,1 |
| SymRepair | Hexyldecanol, Bisabolol, Cetylhydroxyproline Palmitamide, Stearic Acid, Brassica Campestris (Rapeseed) Sterols | | 1 | | 3 | | | | | | | |
| SymSitive 1609 | Pentylene Glycol, 4-t-Butylcyclohexanol | | | 0,5 | | | | | | 0,5 | | |
| SymVital | Aloe Barbadensis Leaf Juice Powder, Magnesium Ascorbyl Phosphate, Rubus Idaeus (Raspberry) Leaf Extract | | | | | 0,1 | | | 0,5 | | | |
| SymWhite 377 | Phenylethyl Resorcinol | | | | | | | | | 0,5 | | |
| Talkum | Talc | | | | | | | | 3 | | | |
| Tamasterol | Phytosterols | | | | | | | | | | 0,3 | |
| Tapioca Pure | Tapioca Starch | | | | | | | | | | | 5 |
| Tego Betain L7 non preserved | Cocamidopropyl Betain | 8 | | | | | | | | | | |
| Tegosoft PC 31 | Poyglyceryl-3 Caprate | | | | | | | | | | 0,3 | |
| Triethanolamin, 99% | Triethanolamine | | | 0,3 | | | | | | | | |
| Vitamin A Palmitat | Retinyl Palmitate | | 0,05 | | | | | | | | | |
| Vitamin E Acetat | Tocopheryl Acetate | | 0,5 | | 0,2 | | | | | | 0,3 | 0,5 |
| Wasser | Water (Aqua) | ad 100 | | ad 100 | ad 100 | ad 100 | ad 100 | ad 100 | ad 100 | ad 100 | ad 100 | ad 100 |

Beispiel 12: Augenkonturen Emulsion, SPF15

| Inhaltsstoffe | INCI-Name | Gew.-% |
|---|---|---|
| Gleditsia triacanthos Holzextrakt (Trockenextraktgehalt 3%) | Propylenglycol, Water (Aqua), Gleditsia triacanthos Extract | 1 |
| Betulin 90% | Betulin | 0,15 |
| Biotive L-Arginine | Arginine | 0,9 |
| Carbopol Ultrez-21 | Acrylates/C10-30 Alkyl Acrylates Crosspolymer | 0,5 |
| Corapan TQ | Diethylhexyl 2,6-Naphthalate | 3 |
| Dracorin GOC | Glyceryl Oleate Citrate, Caprylic/Capric Triglyceride | 1,5 |
| EDTA BD | Disodium EDTA | 0,1 |
| Hydrolite-5 | Pentylene Glycol | 5 |
| Isoadipate | Diisopropyl Adipate | 1 |
| Keltrol CG RD | Xanthan Gum | 0,2 |
| Neo Heliopan 357 | Butylmethoxydibenzoylmethane | 3 |
| Neo Heliopan HMS | Homosalate | 5 |
| Neo Heliopan Hydro, 20%ige Lösung, neutralisiert mit Arginin | Aqua, Phenylbenzimidazole, Sulphonic Acid, Arginin | 10 |
| Neo Heliopan OS | Ethylhexyl Salicylate | 5 |
| SymFinity 1298 | Echinacea Purpurea Extract | 0,1 |
| SymHelios 1031 | Benzylidene Dimethoxydimethylindanone | 0,5 |
| SymMollient L | Neopentyl Glycol Diisononanoate | 2 |
| SymMollient S | Cetearyl Nonanoate | 1 |
| Tegosoft TN | C12-C15 Alkyl benzoate | 5 |
| Vitamin E Acetat | Tocopheryl Acetate | 0,5 |
| Wasser | Water (Aqua) | ad 100 |

**Beispiele: 13 -15: Oral konsumierbare Zubereitungen**

Beispiel 13: Fruchtgummis

| Inhaltsstoffe | Gew.-% |
|---|---|
| Wasser | Ad 100 |
| Saccharose | 34,50 |
| Glucosesirup, DE 40 | 31,89 |
| Iso Syrup C* Tru Sweet 01750 (Cerestar GmbH) | 1,50 |
| Gelatine 240 Bloom | 8,20 |
| Gelbe und rote Lebensmittelfarbstoffe | 0,01 |
| Citronensäure | 0,20 |

(fortgesetzt)

| Inhaltsstoffe | Gew.-% |
|---|---|
| Gleditsia triacanthos Holzextrakt | 0,075 |

Beispiel 14: Zum Direktverzehr geeignete Gelatinekapseln

| Inhaltsstoffe | Gew.-% | | |
|---|---|---|---|
| | I | II | III |
| Gelatinehülle: | | | |
| Glycerin | 2,014 | 2,014 | 2,014 |
| Gelatine 240 Bloom | 7,91 | 7,91 | 7,91 |
| Aspartam | 0,05 | - | - |
| Sucralose | 0,035 | 0,050 | 0,070 |
| Allura Red (roter Farbstoff) | 0,006 | 0,006 | 0,006 |
| Brillant Blue (blauer Farbstoff) | 0,005 | 0,005 | 0,005 |
| | | | |
| Kernzusammensetzung: | | | |
| Pflanzenöltriglyceride (Kokonussölfraktion; coconut oil fraction) | Ad 100 | Ad 100 | Ad 100 |
| Aroma G | 9,95 | 12,0 | 12,0 |
| Gleditsia triacanthos Holzextrakt | | 0,05 | |
| Gleditsia triacanthos Holzextrakt (Pflanzenöl : Trockenextrakt = 90 : 10) | 3 | | 0,5 |

Aroma G hatte dabei folgende Zusammensetzung (Angaben jeweils in Gew.-%): 0,1% Neotam Pulver, 29,3% Pfefferminzöl arvensis, 29,35% Pfefferminz piperita Öl Willamette, 2,97% Sucralose, 2,28% Triacetin, 5,4% Diethyltartrat, 12,1% Pfefferminzöl yakima, 0,7% Ethanol, 3,36% 2-Hydroxyethylmenthylcarbonat, 3,0% 2-Hydroxypropylmenthylcarbonat, 5,77% D-Limonen, 5,67% L-Menthylacetat.

Die zum Direktverzehr geeigneten Gelatinekapseln I, II, III wurden gemäß WO 2004/050069 hergestellt und hatten jeweils einen Durchmesser von 5 mm, das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf.

Beispiel 15: Komprimate in runder Tablettenform

| Inhaltsstoffe | Gew.-% | | |
|---|---|---|---|
| | I | II | III |
| Magnesiumstearat | 0,9 | 0,9 | 0,9 |
| Citronensäure | 0,2 | 0,2 | 0,2 |
| Gleditsia triacanthos Holzextrakt (Maltodextrin : Trockenextrakt = 95 : 5) | 1 | | 5 |
| Gleditsia triacanthos Holzextrakt | | 0,01 | |
| Dextrose | Ad 100 | Ad 100 | Ad 100 |

**Beispiele 16-21: Mundpflegeprodukte**

Beispiel 16: Gelzahncreme

| **Inhaltsstoffe** | I (%) | II (%) | III (%) |
|---|---|---|---|
| Natriumcarboxymethylcellulose | 0,40 | 0,40 | 0,40 |
| Sorbitol 70 %, in Wasser | 72,00 | 72,00 | 72,00 |
| Polyethylenglykol (PEG) 1500 | 3,00 | 3,00 | 3,00 |
| Natriumsaccharinat | 0,07 | 0,07 | 0,07 |
| Natriumfluorid | 0,24 | 0,24 | 0,24 |
| p-Hydroxybenzoesäure (PHB) ethylester | 0,15 | 0,15 | 0,15 |
| Pfefferminzaroma | 1,00 | 1,00 | 1,00 |
| Gleditsia triacanthos Holzextrakt | 0,10 | 0,02 | 0,001 |
| Abrasive Kieselsäure | 11,00 | 11,00 | 11,00 |
| Verdickende Kieselsäure | 6,00 | 6,00 | 6,00 |
| Natriumdodecylsulfat | 1,40 | 1,40 | 1,40 |
| Dest. Wasser | ad 100 | ad 100 | ad 100 |

Beispiel 17: Zahncreme gegen Plaque

| **Inhaltsstoffe** | I (%) | II (%) | III (%) |
|---|---|---|---|
| Carrageenan | 0,90 | 0,90 | 0,90 |
| Glycerin | 15,00 | 15,00 | 15,00 |
| Sorbitol 70 %, in Wasser | 25,00 | 25,00 | 25,00 |
| PEG 1000 | 3,00 | 3,00 | 3,00 |
| Natriumfluorid | 0,24 | 0,24 | 0,24 |
| Tetrakaliumdiphosphat | 4,50 | 4,50 | 4,50 |
| Tetranatriumdiphosphat | 1,50 | 1,50 | 1,50 |
| Natriumsaccharinat | 0,40 | 0,40 | 0,40 |
| Fällungskieselsäure | 20,00 | 20,00 | 20,00 |
| Titandioxid | 1,00 | 1,00 | 1,00 |
| PHB Methylester | 0,10 | 0,10 | 0,10 |
| Grünes Minzaroma (Spearmint) | 1,10 | 1,10 | 1,10 |
| Gleditsia triacanthos Holzextrakt | 0,005 | 0,50 | 0,10 |
| Natriumdodecylsulfat | 1,30 | 1,30 | 1,30 |
| Dest. Wasser | ad 100 | ad 100 | ad100 |

Beispiel 18: Zahncreme für empfindliche Zähne

| **Inhaltsstoffe** | I (%) | II (%) | III (%) |
|---|---|---|---|
| Natriumcarboxymethylcellulose | 0,70 | 0,70 | 0,70 |
| Xanthan gum | 0,50 | 0,50 | 0,50 |
| Glycerin | 15,00 | 15,00 | 15,00 |
| Sorbitol 70 %, in Wasser | 12,00 | 12,00 | 12,00 |
| Kaliumnitrat | 5,00 | 5,00 | 5,00 |
| Natriummonofluorophosphat | 0,80 | 0,80 | 0,80 |
| PHB Methylester | 0,15 | 0,15 | 0,15 |
| PHB Propylester | 0,05 | 0,05 | 0,05 |
| Natriumsaccharinat | 0,20 | 0,20 | 0,20 |
| Eucalyptus/Menthol Aroma | 1,00 | 1,00 | 1,00 |
| Gleditsia triacanthos Holzextrakt | 0,03 | 0,30 | 0,006 |
| Calciumcarbonat | 35,00 | 35,00 | 35,00 |
| Siliciumdioxid | 1,00 | 1,00 | 1,00 |
| Natriumdodecylsulfat | 1,50 | 1,50 | 1,50 |
| Dest. Wasser | ad 100 | ad 100 | ad 100 |

Beispiel 19: Mundwasser mit Fluorid

| **Inhaltsstoffe** | I (%) | II (%) | III (%) |
|---|---|---|---|
| Ethanol | 7,00 | 7,00 | 7,00 |
| Glycerin | 12,00 | 12,00 | 12,00 |
| Natriumfluorid | 0,05 | 0,05 | 0,05 |
| Pluronic F-127® (BASF) | 1,40 | 1,40 | 1,40 |
| Natriumphosphatpuffer pH 7.0 | 1,10 | 1,10 | 1,10 |
| Sorbinsäure | 0,20 | 0,20 | 0,20 |
| Natriumsaccharinat | 0,10 | 0,10 | 0,10 |
| Zimt/Menthol Aroma | 0,15 | 0,15 | 0,15 |
| Gleditsia triacanthos Holzextrakt | 0,002 | 0,05 | 0,80 |
| Farbstoff | 0,01 | 0,01 | 0,01 |
| Dest. Wasser | ad 100 | ad 100 | ad 100 |

Beispiel 20: Kaugummi

| **Inhaltsstoffe** | I (%) | II (%) | III (%) |
|---|---|---|---|
| Kaugummibase | 21,00 | 21,00 | 21,00 |
| Glukosesirup | 16,50 | 16,50 | 16,50 |
| Glycerin | 0,50 | 0,50 | 0,50 |

(fortgesetzt)

| **Inhaltsstoffe** | I (%) | II (%) | III (%) |
|---|---|---|---|
| Puderzucker | ad 100 | ad 100 | ad 100 |
| Minzaroma | 1,50 | 1,50 | 1,50 |
| Gleditsia triacanthos Holzextrakt | 0,05 | 0,002 | 0,30 |

Beispiel 21: Zuckerfreies Kaugummi

| **Inhaltsstoffe** | I (%) | II (%) | III (%) |
|---|---|---|---|
| Kaugummibase | 30,00 | 30,00 | 30,00 |
| Sorbitol, Pulver | ad 100 | ad 100 | ad 100 |
| Palatinit | 9,50 | 9,50 | 9,50 |
| Xylitol | 2,00 | 2,00 | 2,00 |
| Mannitol | 3,00 | 3,00 | 3,00 |
| Aspartam | 0,10 | 0,10 | 0,10 |
| Acesulfam K | 0,10 | 0,10 | 0,10 |
| Emulgum / Emulgator | 0,30 | 0,30 | 0,30 |
| Sorbitol 70 %, in Wasser | 14,00 | 14,00 | 14,00 |
| Glycerin | 1,00 | 1,00 | 1,00 |
| Zimt/Mentholaroma | 1,50 | 1,50 | 1,50 |
| Gleditsia triacanthos Holzextrakt | 0,003 | 1,0 | 0,20 |

**Patentansprüche**

**1.** Nicht-therapeutische Verwendung eines Holzextraktes von *Gleditsia triacanthos*

i) zur Vorbeugung, Behandlung oder Verringerung von Cellulite,
und/oder
(ii) zum nicht-therapeutischen

- Hemmen der Differenzierung von Preadipozyten,
und/oder
- Hemmen der Lipogenese in Adipozyten,
und/oder
- Reduzieren der in subkutanem Fettgewebe enthaltenen Lipidmenge.

**2.** Nicht-therapeutische Verwendung nach Anspruch 1, wobei der Holzextrakt ein Extrakt des Kernholzes von *Gleditsia triacanthos* ist.

**3.** Mischung enthaltend

(a) einen Holzextrakt
hergestellt oder herstellbar mit einem Verfahren umfassend oder bestehend aus den Schritten:

(1) Bereitstellen von Holz, vorzugsweise von Holzraspat, von *Gleditsia triacanthos,*
(2) Versetzen des in Schritt (1) bereitgestellten Holzes mit einem Extraktionsmittel ausgewählt aus der

Gruppe bestehend aus Wasser, Methanol, Ethanol, n-Propanol, Isopropanol, Aceton, Essigester, überkritischem Kohlendioxid und Mischungen zweier oder mehrerer dieser Extraktionsmittel,
(3) bis zu 24 h Extrahieren des in Schritt (2) mit dem Extraktionsmittel versetzten Holzes zum Gewinnen eines Holzextraktes,
und
(4) gegebenenfalls partielles oder vollständiges Entfernen des oder der in Schritt (2) verwendeten Extraktionsmittel,

und
(b) ein oder mehrere mehrwertige Alkohole mit 3 oder 4 C-Atomen, vorzugsweise ausgewählt aus der Gruppe bestehend aus 1,2-Propandiol, 1,3-Propandiol, 1,2-Butandiol, 1,3-Butandiol, 1,4-Butandiol und Glycerin, sowie gegebenenfalls zusätzlich
(c) Wasser
und/oder
(d) Ethanol,
und
(e) einem oder mehreren Konservierungsmitteln gewählt aus der Gruppe bestehend aus Benzoesäure, ihre Ester und Salze, Propionsäure und ihre Salze, Salicylsäure und ihre Salze, 2,4-Hexadiensaeure (Sorbinsäure) und ihre Salze, Ester der p-Hydroxybenzoesäure (Parabene), Formaldehyd und Paraformaldehyd, 2-Hydroxybiphenylether und seine Salze, 2-Zinksulfidopyridin-N-oxid, anorganische Sulfite und Bisulfite, Natriumiodat, Chlorbutanolum, 4-Ethylquecksilber-(II)5-Amino-1,3-bis(2-Hydroxybenzoesaeure, ihre Salze und Ester, Dehydracetsäure, Ameisensäure, 1,6-Bis(4-amidino-2-bromphenoxy)-n-hexan und seine Salze, das Natriumsalz der Ethylqueck-silber-(II)-thiosalicylsäure, Phenylquecksilber und seine Salze, 10-Undecylensaeure und ihre Salze, 5-Amino-1,3-bis(2-ethylhexyl)-5-methyl-hexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxydiphenylether, 4-Chlor-3,5-dimethylphenol, 1,1'-Methylen-bis(3-(1-hydroxymethyl-2,4-dioximidazolidin-5-yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1-(4-Chlorphenoxy)1(1H-imidazol-1-yl)-3,3-dimethyl-2-butanon, 1,3-Bis-(hydroxymethyl)-5,5-dimethyl-2,4-imidazolidindion, Benzylalkohol, Octopirox, 1,2-Dibrom-2,4-dicyanobutan, 2,2'-Methylen-bis(6-brom-4-chlorphenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxypropan-2-ol, N-Alkyl($C_{12}$-$C_{22}$)trimethyl-ammoniumbromid und -chlorid, 4,4-Dimethyl-1,3-oxazolidin, N-Hydroxymethyl-N-(1,3-di(hydroxymethyl)-2,5-dioxoimidazolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-($C_8$-$C_{18}$)-dimethyl-benzyl-ammoniumchlorid, Alkyl-($C_8$-$C_{18}$)-dimethyl-benzylammonium-bromid, Alkyl-($C_8$-$C_{18}$)-dimethyl-benzyl-ammoniumsaccharinat, Benzylhemiformal, 3-lod-2-propinyl-butylcarbamat, Natrium-hydroxymethyl-aminoacetat oder Natriumhydroxymethylaminoacetat,
wobei die Gesamtmenge an Konservierungsmittel vorzugsweise im Bereich von 0.05 bis 5 Gew.% bezogen auf das Gesamtgewicht der Mischung ist.

**4.** Nicht-therapeutische Verwendung einer kosmetischen, dermatologischen oder pharmazeutischen Zubereitung, enthaltend einen Holzextrakt von *Gleditsia triacanthos,* zur Vorbeugung, Behandlung oder Verringerung von Cellulite.

**5.** Dermatologische oder pharmazeutische Zubereitung, enthaltend einen Holzextrakt von *Gleditsia triacanthos,* in einer ausreichenden Menge zur Verwendung als Medikament zum

- Hemmen der Differenzierung von Preadipozyten,
und/oder
- Hemmen der Lipogenese in Adipozyten,
und/oder
- Reduzieren der in subkutanem Fettgewebe enthaltenen Lipidmenge.

**6.** Verwendung nach Anspruch 4 oder Zubereitung nach Anspruch 5, wobei die Zubereitung eine Mischung nach Anspruch 3 enthält.

**7.** Verwendung nach einem der Ansprüche 4 oder Zubereitung nach Anspruch 5, wobei der Holzextrakt ein Extrakt

des Kernholzes von *Gleditsia triacanthos* ist.

**8.** Verwendung nach einem der Ansprüche 4, 6 oder 7 oder Zubereitung nach Anspruch 5, **dadurch gekennzeichnet, dass** der Holzextrakt Fustin, vorzugsweise Fustin und Fisetin, enthält.

**9.** Verwendung nach einem der Ansprüche 4 oder 6 bis 8 oder Zubereitung nach Anspruch 5, wobei die Zubereitung ferner ein, zwei, drei oder mehrere Lipolyse-Stimulantien umfasst, wobei einer, mehrere oder alle der Lipolyse-Stimuantien

- eine Verbindung der Formel (Xa) oder ein pharmazeutisch akzeptables Salz einer solchen Verbindung ist,

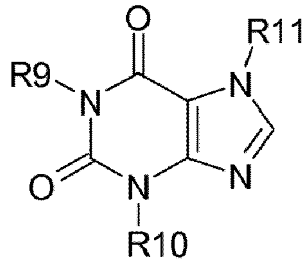

(Xa)

wobei R9, R10 und R11 unabhängig voneinander Wasserstoff oder Methyl bedeuten, und/oder
- eine Verbindung der Formel (PhEA) oder ein pharmazeutisch akzeptables Salz einer solchen Verbindung ist,

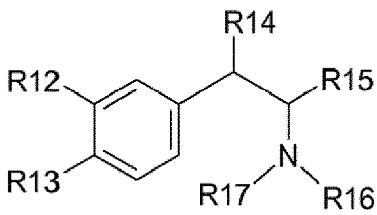

(PhEA)

wobei, unabhängig voneinander,

R12 und R13 jeweils Wasserstoff, Hydroxy und Methoxy,
R14 Wasserstoff, Hydroxy und Methyl,
R15 Wasserstoff und Methyl und
R16 und R17 jeweils Wasserstoff und C1-C4-Alkyl bedeuten.

**10.** Verwendung bzw. Zubereitung nach 9, wobei eine, mehrere oder alle Verbindungen der Formel (PhEA) Verbindungen der Formel (PhEA-i) und deren pharmazeutisch akzeptable Salze sind,

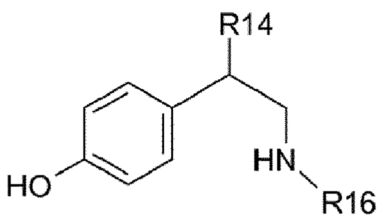

(PhEA-i)

wobei der Rest R14 Wasserstoff, Hydroxy und Methyl und der Rest R16 Wasserstoff und C1-C4-Alkyl bedeuten.

**11.** Verwendung nach einem der Ansprüche 4 oder 6 bis 10 oder Zubereitung nach Anspruch 5, wobei die Gesamtmenge an Holzextrakt, bezogen auf die Masse des Trockenextraktes, 0,00001 bis 10 Gew.-%, vorzugsweise 0,0001 bis 5 Gew.-%, bevorzugt 0,001 bis 2 Gew.-%, weiter bevorzugt 0,001 bis 1 Gew.-%, beträgt, jeweils bezogen auf die gesamte Zubereitung.

**12.** Verwendung oder Zubereitung nach einem der Ansprüche 9 bis 11, wobei die Gesamtmenge der Verbindungen der Formel (Xa) 0,005 - 10 Gew.-%, vorzugsweise 0,05 - 5 Gew.-% und besonders bevorzugt 0,5- 2,5 Gew.-% beträgt.

**Claims**

**1.** Non-therapeutic use of a wood extract of *Gleditsia triacanthos*

(i) for preventing, treating or reducing cellulite,
and/or
(ii) for non-therapeutic

- inhibiting the differentiation of preadipocytes,
and/or
- inhibiting lipogenesis in adipocytes,
and/or
- reducing the amount of lipids in subcutaneous fatty tissue.

**2.** Non-therapeutic use according to claim 1, wherein the wood extract is an extract of the heartwood of *Gleditsia triacanthos.*

**3.** Mixture comprising

(a) a wood extract
obtained or obtainable by a method comprising or consisting of the following steps:

(1) providing wood, preferably holzraspat, of *Gleditsia triacanthos,*
(2) adding one or more extractants selected from the group consisting of water, methanol, ethanol, n-propanol, isopropanol, acetone, ethyl acetate, super critical carbon dioxide and mixtures thereof to the wood of (1),
(3) obtaining a wood extract for up to 24 h from the wood of (2) and optionally
(4) partially or completely removing the one or more extractants used in step (2),

and
(b) one or more polyhydric alcohols having 3 or 4 carbon atoms, preferably selected from the group consisting of 1,2-propanediol, 1,3-propanediol, 1,2-butanediol, 1,3-butanediol, 1,4-butanediol and glycerin,
and optionally
(c) water
and/or
(d) ethanol,
and
(e) one or more preservatives selected from the group consisting of benzoic acid, its esters and salts, propionic acid and its salts, salicylic acid and its salts, 2,4-hexadienoic acid (sorbic acid) and its salts, esters of p-hydroxybenzoic acid (parabens), formaldehyde and paraformaldehyde, 2-hydroxybiphenyl ether and its salts, 2 Zinc sulfide-pyridine N-oxide, inorganic sulphites and bisulphites, sodium iodate, chlorobutanol, 4-ethylmercury- (II) 5-amino-1,3-bis (2-hydroxybenzoic acid, its salts and esters, Dehydroacetic acid, formic acid, 1,6-bis (4-amidino-2-bromophenoxy) -n-hexane and its salts, the sodium salt of ethylmercury- (II) thiosalicylic acid, phenyl mercury and its salts, 10-Undecylen¬ acid and its salts, 5-amino-1,3-bis (2-ethylhexyl) -5-methylhexahydropyrimidine, 5-bromo-5-nitro-1,3-dioxane, 2-bromo-2-nitro- 1,3-propanediol, 2,4-dichlorobenzyl alcohol, N- (4-chlorophenyl) -N '- (3,4-dichlorophenyl) -urea, 4-chloro-m-cresol, 2,4,4'- Trichloro-2'-hydroxy-diphenyl ether, 4-chloro-3,5-dimethylphenol, 1,1'-methylene-bis (3- (1-hydro xymethyl-2,4-dioximidazolidin-5-yl) urea), poly (hexamethylenediguanide) hydrochloride, 2-phenoxyethanol, hexamethylenetetramine, 1- (3-chloroallyl) -3,5,7-triaza-1 -azonia-adamantane chloride, 1- (4-chlorophenoxy) -1 (1H-imidazol-1-yl) -3,3-dimethyl-2-butanone, 1,3-bis (hydroxymethyl) -5,5 -dimethyl-2,4-imidazolidinedione, benzyl alcohol, octopirox, 1,2-dibromo-2,4-dicyanobutane, 2,2'-methylenebis (6-bromo-4-chlorophenol), bromochlorophene, mixture of 5-chloro 2-methyl-3 (2H) -isothiazolinone and 2-methyl-3 (2H) isothiazolinone with magnesium chloride and magnesium nitrate, 2-benzyl-4-chlorophenol, 2-chloroacetamide, chlorhexidine, chlorhexidine acetate, chlorhexidine gluconate, Chlorhexidine hydrochloride, 1-phenoxypropan-2-ol, N-alkyl (C12-C22) trimethylammonium bromide and chloride, 4,4-dimethyl-1,3-oxazolidine, N-hydroxy-methyl-N-(1,3-) di (hydroxymethyl) -2,5-dioxoimidazolidin-4-yl) -N'-hydroxymethylthiourea, 1,6-bis (4-amidino-phenoxy) -n-hexane and its salts, glutaraldehyde , 5-ethyl-1-aza-3,7-dioxabicyclo (3.3.0) octane, 3- (4-chlorophenoxy) -1,2-propanediol, hyamine, alkyl (C8-C18) dimethyl benzyl ammonium chloride, alkyl (C8-C18) dimethyl benzyl ammonium bromide, alkyl (C8 -C 18) -dimethylbenzylammonium saccharinate, benzylhemiformal, 3-iodo-2-propynyl-butylcarbamate, sodium hydroxymethylaminoacetate or sodium hydroxymethylaminoacetate,
wherein the total amount of preservatives is preferably in the range of 0.05 to 5 % wt based on the total weight of the mixture.

**4.** Non-therapeutic use of a cosmetic, dermatological or pharmaceutical preparation comprising a wood extract of *Gleditsia triacanthos* for preventing, treating, or reducing cellulite.

**5.** Dermatological or pharmaceutical preparation, comprising a wood extract of *Gleditsia triacanthos* in a sufficient amount for use as a medicament for

- inhibiting the differentiation of preadipocytes,
and/or
- inhibiting lipogenesis in adipocytes,
and/or
- reducing the amount of lipids in subcutaneous fatty tissue.

**6.** Use according to claim 4 or preparation according to claim 5, wherein the preparation comprises a mixture according to claim 3.

**7.** Use according to claim 4 or preparation according to claim 5, wherein the wood extract is an extract of the heartwood of *Gleditsia triacanthos.*

**8.** Use according to any of the claims 4, 6 or 7 or preparation according to claim 5, **characterized in that** the wood extract comprises fustin, preferably fustin and fisetin.

**9.** Use according to any of the claims 4 or 6 to 8 or preparation according to claim 5, wherein the preparation comprises further one, two, three or more lipolysis stimulants, wherein one, more or all lipolysis stimulants

- is a compound of Formula (Xa) or a pharmaceutically acceptable salt thereof,

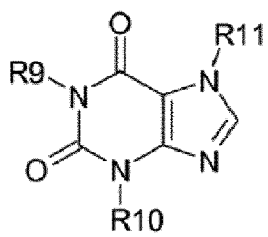

(Xa)

wherein R9, R10 and R11 are independently from each other hydrogen or methyl, and/or
- is a compound of Formula (PhEA)) or a pharmaceutically acceptable salt thereof,

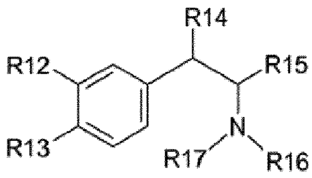

(PhEA)

wherein, independently from each other,

R12 and R13 each are hydrogen, hydroxy and methoxy,
R14 is hydrogen, hydroxy and methyl,
R15 is hydrogen and methyl and
R16 and R17 are each hydrogen an C1-C4 alkyl.

**10.** Use/Preparation according to claim 9, wherein one, more or all compounds of Formula (PhEA) are compounds of Formula (PhEA-i) or their a pharmaceutically acceptable salts,

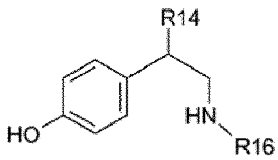

(PhEA-i)

wherein R14 is hydrogen, hydroxyl and methyl and R16 is hydrogen and C1-C4 alkyl.

**11.** Use according to any of claims 4 or 6 to 10 or preparation according to claim 5, wherein the total amount of wood extract, based on the mass of the dry extract, is 0.00001 to 10 % wt, preferably 0.0001 to 5% wt, preferably 0.001 to 2% wt, more preferably 0.001 to 1% wt, each based on the total preparation.

**12.** Use or preparation according to any of claims 9 to 11, wherein the total amount of the compounds of Formula (Xa) is 0.005 to 10% wt, preferably 0.05 to 5% wt and more preferably 0.5 to 2.5% wt.

**Revendications**

**1.** Utilisation non thérapeutique d'un extrait de bois de *Gleditsia triacanthos*

i) pour la prévention, le traitement ou la réduction de la cellulite,
et/ou
ii) pour

- l'inhibition non thérapeutique de la différenciation des pré-adipocytes, et/ou
- l'inhibition non thérapeutique de la lipogenèse dans les adipocytes et/ou
- la réduction non thérapeutique de la quantité de lipides contenue dans les tissus graisseux sous-cutanés.

**2.** Utilisation non thérapeutique selon la revendication 1, dans laquelle l'extrait de bois est un extrait du bois de coeur de *Gleditsia triacanthos.*

**3.** Mélange contenant :

(a) un extrait de bois fabriqué ou fabricable par un procédé comprenant ou constitué par les étapes suivantes :

(1) la préparation de bois, notamment de bois râpé, de *Gleditsia triacanthos,*
(2) le mélange du bois préparé à l'étape (1) avec un agent d'extraction choisi dans le groupe constitué par

l'eau, le méthanol, l'éthanol, le n-propanol, l'isopropanol, l'acétone, l'ester acétique, le dioxyde de carbone supercritique et les mélanges de deux ou plus de ces agents d'extraction,
(3) l'extraction pendant jusqu'à 24 h du bois mélangé avec l'agent d'extraction à l'étape (2) pour obtenir un extrait de bois,
et
(4) éventuellement l'élimination partielle ou totale du ou des agents d'extraction utili sés à l'étape (2),
et

(b) un ou plusieurs alcools polyvalents contenant 3 ou 4 atomes C, de préférence choisis dans le groupe constitué par le 1,2-propanediol, le 1,3-propanediol, le 1,2-butanediol, le 1,3- butanediol, le 1,4-butanediol et la glycérine,
ainsi qu'éventuellement en outre
(c) de l'eau,
et/ou
(d) de l'éthanol,
et
(e) un ou plusieurs conservateurs choisis dans le groupe constitué par l'acide benzoïque, ses esters et sels, l'acide propionique et ses sels, l'acide salicylique et ses sels, l'acide 2,4- hexadiénique (acide sorbique) et ses sels, les esters de l'acide p-hydroxybenzoïque (para bènes), le formaldéhyde et le paraformaldéhyde, l'éther 2-hydroxybiphénylique et ses sels, le N-oxyde de 2-zinc-sulfidopyridine, les sulfites et bisulfites inorganiques, l'iodate de sodium, le chlorobutanol, l'acide 4-éthylmercure-(II)-5-amino-1,3-bis-2-hydroxybenzoïque, ses sels et esters, l'acide déshy-dracétique, l'acide formique, le 1,6-bis(4-amidino-2-bromophénoxy)-n-hexane et ses sels, le sel de sodium de l'acide éthylmercure- (II)-thiosalicylique, le phénylmercure et ses sels, l'acide 10-undécylénique et ses sels, la 5-amino-1,3-bis(2-éthylhexyl)-5-méthyl-hexahydropyrimidine, le 5-bromo-5-nitro-1,3-dioxane, le 2-bromo-2-nitro-1,3-propanediol, l'alcool 2,4-dichlorobenzylique, la N-(4-chlorophényl)-N'-(3,4-dichlorophényl)-urée, le 4-chloro-m-crésol, l'éther 2,4,4'-trichloro-2'- hydroxydiphény-lique, le 4-chloro-3,5-diméthylphénol, la 1,1'-méthylène-bis(3-(1-hydroxyméthyl-2,4-dioximidazolidin-5-yl)urée), le polychlorhydrate d'(hexaméthylène-diguanide), le 2-phénoxyéthanol, l'hexaméthylène-tétramine, le chlorure de 1-(3-chloroallyl)-3,5,7-triaza-1-azonia-adamantane, la 1-(4-chlorophénoxy)1(1H-imidazol-1-yl)-3,3-diméthyl-2-butanone, la 1,3-bis-(hydroxyméthyl)-5,5-diméthyl-2,4-imidazolidinedione, l'alcool ben-zylique, l'octopirox, le 1,2-dibromo-2,4-dicyanobutane, le 2,2'-méthylène-bis(6-bromo-4-chlorophénol), le bromochlorophène, un mélange de 5- chloro-2-méthyl-3(2H)-isothiazolinone et de 2-méthyl-3(2H)isothiazolinone avec du chlo rure de magnésium et du nitrate de magnésium, le 2-benzyl-4-chlorophénol, le 2-chloroacétamide, la chlorhexidine, l'acétate de chlorhexidine, le gluconate de chlorhexidine, le chlorhydrate de chlorhexidine, le 1-phénoxypropan-2-ol, le bromure et le chlorure de N-alkyl($C_{12}$-$C_{22}$)triméthyl-ammonium, la 4,4-diméthyl-1,3-oxazolidine, la N-hydroxyméthyl-N-(1,3-di(hydroxyméthyl)-2,5-dioxoimidazolidin-4-yl)-N'-hydroxy- méthylurée, le 1,6-bis(4-amidino-phénoxy)-n-hexane et ses sels, le glutaraldéhyde, le 5-éthyl-1-aza-3,7-dioxabicyclo(3.3.0)octane, le 3-(4-chlorophénoxy)-1,2-propanediol, l'hyamine, le chlorure d'alkyl-($C_8$-$C_{18}$)-diméthyl-benzyl-ammonium, le bromure d'alkyl-($C_8$-$C_{18}$)-diméthyl-benzyl-ammonium, le saccharinate d'alkyl-($C_8$-$C_{18}$)-diméthyl-benzylammonium, le benzylhémiformal, le carbamate de 3-iodo-2-propynyl-butyle, l'hydroxyméthyl-aminoacétate de sodium ou l'hydroxyméthylaminoacétate de sodium, la quantité totale de conservateur étant de préférence dans la plage allant de 0,05 à 5 % en poids, par rapport au poids total du mélange.

**4.** Utilisation non thérapeutique d'une préparation cosmétique, dermatologique ou pharmaceutique, contenant un extrait de bois de *Gleditsia triacanthos,* pour la prévention, le traitement ou la réduc tion de la cellulite.

**5.** Préparation dermatologique ou pharmaceutique, contenant un extrait de bois de *Gleditsia triacan thos,* en une quantité suffisante pour une utilisation en tant que médicament pour

- l'inhibition de la différenciation des pré-adipocytes,
et/ou
- l'inhibition de la lipogenèse dans les adipocytes
et/ou
- la réduction de la quantité de lipides contenue dans les tissus graisseux sous-cutanés.

**6.** Utilisation selon la revendication 4 ou préparation selon la revendication 5, dans laquelle la préparation contient un mélange selon la revendication 3.

**7.** Utilisation selon la revendication 4 ou préparation selon la revendication 5, dans laquelle l'extrait de bois est un extrait du bois de coeur de *Gleditsia triacanthos.*

**8.** Utilisation selon l'une quelconque des revendications 4, 6 ou 7 ou préparation selon la revendication 5, **caractérisée en ce que** l'extrait de bois contient de la fustine, de préférence de la fustine et de la fisétine.

**9.** Utilisation selon l'une quelconque des revendications 4 ou 6 à 8 ou préparation selon la revendication 5, dans laquelle la préparation comprend en outre un, deux, trois stimulants de la lipolyse ou plus, un, plusieurs ou tous les stimulants de la lipolyse

- étant un composé de formule (Xa) ou un sel pharmaceutiquement acceptable d'un tel composé

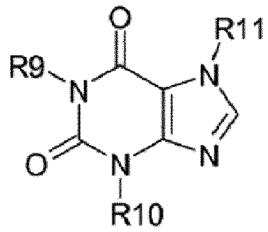

(Xa)

dans laquelle R9, R10 et R11 signifient indépendamment les uns des autres hydrogène ou méthyle, et/ou

- étant un composé de formule (PhEA) ou un sel pharmaceutiquement acceptable d'un tel composé

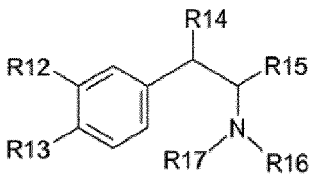

(PhEA)

dans laquelle, indépendamment les uns des autres,

R12 et R13 signifient chacun hydrogène, hydroxy et méthoxy,
R14 signifie hydrogène, hydroxy et méthyle,
R15 signifie hydrogène et méthyle, et
R16 et R17 signifient chacun hydrogène et alkyle en C1-C4.

**10.** Utilisation ou préparation selon la revendication 9, dans laquelle un, plusieurs ou tous les composés de formule (PhEA) sont des composés de formule (PhEA-i) et leurs sels pharmaceutiquement

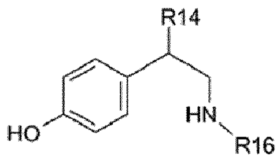

(PhEA-i)

accep tables dans laquelle le radical R14 signifie hydrogène, hydroxy et méthyle, et le radical R16 signifie hydro gène et alkyle en C1-C4.

**11.** Utilisation selon l'une quelconque des revendications 4 ou 6 à 10 ou préparation selon la revendication 5, dans

laquelle la quantité totale d'extrait de bois, par rapport à la masse de l'extrait sec, est de 0,00001 à 10 % en poids, avantageusement de 0,0001 à 5 % en poids, de préférence de 0,001 à 2 % en poids, de manière davantage préférée de 0,001 à 1 % en poids, à chaque fois par rapport à l'ensemble de la préparation.

**12.** Utilisation ou préparation selon l'une quelconque des revendications 9 à 11, dans laquelle la quantité totale des composés de formule (Xa) est de 0,005 à 10 % en poids, de préférence de 0,05 à 5 % en poids et de manière particulièrement préférée de 0,5 à 2,5 % en poids.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1234572 A
- DE 102004032837
- WO 2010048114 A
- WO 2005002672 A
- FR 2067649
- GB 1065910 A
- CN 101317960
- CN 101224287
- JP 2001288047 B
- JP 2000044439 B
- JP 2001131026 B
- US 20060089413 A
- WO 2008046791 A
- WO 2005123101 A
- WO 2008046795 A
- WO 2007042472 A
- US 20080070825 A
- WO 0176572 A
- WO 0215686 A
- WO 2008046676 A
- WO 2006053912 A
- WO 2007110415 A
- WO 2005107692 A
- WO 2006015954 A
- WO 2007128723 A
- WO 2007060256 A
- WO 2006045760 A
- EP 2168570 A
- EP 2193785 A
- US 20050136537 A
- WO 2005123101 A1
- WO 02069992 A
- US 4150052 A
- WO 2005049553 A
- WO 2004026840 A
- WO 2004050069 A

### In der Beschreibung aufgeführte Nicht-Patentliteratur

- *Journal of Biochemistry,* 2004, vol. 135 (1), 85-91
- *Laut Biochem. Pharmacol.,* 1992, vol. 44, 1307-1315
- *Chem. Biol. Drug Des.,* 2009, vol. 74, 619-624
- *Int. J. Mol. Med.,* 2009, vol. 23 (1), 121-9
- *Yakugaku Zasshi,* 1957, vol. 77, 1208-1210
- *Compt. Rend. Acad. Sci.,* 1955, vol. 240, 1362-1364
- *Yakugaku Zasshi,* 1957, vol. 77
- **S. ARCTANDER.** Perfume and Flavor Chemicals. Eigenverlag, 1969
- Surburg, Panten, Common Fragrance and Flavor Materials. Wiley-VCH, 2006
- *Naunyn-Schmiedeberg's Archives of Pharmacology,* 1999, vol. 359, 310-321